(19) Europäisches Patentamt / European Patent Office / Office européen des brevets

(11) **EP 4 176 258 B1**

(12) **EUROPEAN PATENT SPECIFICATION**

(45) Date of publication and mention of the grant of the patent:
**22.07.2026 Bulletin 2026/30**

(21) Application number: **20943234.3**

(22) Date of filing: **01.07.2020**

(51) International Patent Classification (IPC):
*G01N 33/24* (2006.01)    *E21B 49/00* (2006.01)
*E21B 47/00* (2012.01)    *G01V 1/40* (2006.01)
*G01V 1/50* (2006.01)    *G01V 20/00* (2024.01)
*E21B 41/00* (2006.01)

(52) Cooperative Patent Classification (CPC):
**G01V 20/00; E21B 41/0035; E21B 49/005;**
G01N 33/24; G01V 1/50

(86) International application number:
**PCT/US2020/040539**

(87) International publication number:
**WO 2022/005475 (06.01.2022 Gazette 2022/01)**

(54) **STRATIGRAPHIC METHODS**

STRATIGRAPHISCHE VERFAHREN

PROCÉDÉS STRATIGRAPHIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.05.2023 Bulletin 2023/19**

(73) Proprietor: **Chevron U.S.A. Inc.**
**San Ramon, CA 94583 (US)**

(72) Inventors:
- **MONTGOMERY, Paul**
 **Bricklehampton, Pershore WR10 3HQ (GB)**
- **RATCLIFFE, Kenneth, Thomas**
 **Powys SY21 9BW (GB)**
- **MATHIA, Eliza**
 **Birmingham, West Midlands B15 2EX (GB)**
- **WIGAND, Marcus, Oliver**
 **Missouri City, TX 77459 (US)**

(74) Representative: **Haseltine Lake Kempner LLP**
**One Portwall Square**
**Portwall Lane**
**Bristol BS1 6BH (GB)**

(56) References cited:
WO-A1-2015/070022    WO-A1-2019/204555
US-A1- 2013 144 561    US-A1- 2014 100 833
US-A1- 2015 310 140    US-A1- 2016 018 556
US-A1- 2016 018 556    US-A1- 2018 364 381
US-A1- 2019 339 248

- **OU CHENGHUA ET AL: "Fine reservoir structure modeling based upon 3D visualized stratigraphic correlation between horizontal wells: methodology and its application", JOURNAL OF GEOPHYSICS AND ENGINEERING, INSTITUTE OF PHYSICS PUBLISHING, BRISTOL, GB, vol. 14, no. 6, 22 November 2017 (2017-11-22), pages 1557 - 1571, XP020322167, ISSN: 1742-2140, [retrieved on 20171122], DOI: 10.1088/1742-2140/ AA871E**

**(Cont. next page)**

- MOHSEN ABDUL ET AL: "Horizontal Well Correlation Using Real Time Data and Log Prediction in Geosteering Complex Reservoirs of Saudi Arabia", 13 March 2013 (2013-03-13), XP093247978, Retrieved from the Internet <URL:https://watermark.silverchair.com/spe-164151-ms.pdf?token=AQECAHi208BE49Ooan9kkhW_Er cy7Dm3ZL_9Cf3qfKAc485ysgAAA28wggNrBgkq hkiG9w0BBwagggNcMIIDWAIBADCCA1EGCSq GSIb3DQEHATAeBglghkgBZQMEAS4wEQQMe1 m8NgMCJDYuKDINAgEQgIIDIthxyQEBkASipTi d9MZ_A6zAc7LORY4N4T2EMBO7X6nLwrM4ma AtO5KoFiEJRNN6L6Y7HwvMQRfofW8HAR2d2a> [retrieved on 20250328]

- EL GEZEERY T ET AL: "Innovative Geosteering Technology Utilized in Drilling Smart Multi-lateral Wells, Kuwait", OFFSHORE TECHNOLOGY CONFERENCE, 6 September 2013 (2013-09-06), XP093264783, [retrieved on 20250328]

## Description

### Field

The present disclosure concerns methods for use in stratigraphy, associated computer programs, methods for measuring isotopic signatures of rock samples, and methods of dating rock samples.

### Background

Stratigraphic methods are a subclass of geological methods used to identify correlations between subterranean rocks in a region such as a sedimentary basin. Stratigraphic models can enable a geologist to gain an understanding of the geological processes which have shaped such a region. Such stratigraphic models are useful in the field of hydrocarbon exploration because they enable a hydrocarbon explorer to more accurately target locations in a petroliferous basin at which hydrocarbon deposits (and particularly of the desired type and size) are likely to be found. As conventional hydrocarbon sources become exhausted, there is a greater focus on extracting hydrocarbons from unconventional sources such as shale rocks and tight rock formations. As unconventional hydrocarbon extraction methods (such as hydraulic fracturing) can be more complex and expensive to implement than conventional hydrocarbon extraction methods, there is an increased need to target desirable hydrocarbon deposits more easily and more accurately. Improved stratigraphic methods would therefore be of benefit.

### Summary

According to a first aspect, a method comprises determining (e.g. identifying) stratigraphic correlations between two or more sets of stratigraphic measurements according to claim 1.

Each set of stratigraphic measurements may derive from (i.e. have been obtained from) a different well in a region. For example, each set of stratigraphic measurements may derive from (i.e. may be a set of stratigraphic measurements obtained from) a different lateral well in the region.

The (e.g. lateral) well may be a (e.g. lateral) hydrocarbon well, i.e. a (e.g. lateral) well used for hydrocarbon exploration or extraction. For example, the (e.g. lateral) well may be a (e.g. lateral) oil well or a (e.g. lateral) gas well.

It will be appreciated that wells can typically be divided into vertical wells and lateral wells. Vertical wells are of the type commonly used in conventional hydrocarbon exploration and are substantially vertical in alignment. That is to say, vertical wells extend substantially vertically downwards below the ground surface. In contrast, lateral wells are of the type commonly used in unconventional hydrocarbon exploration, for example in the extraction of hydrocarbons from unconventional sources (such as oil shales or tight rock formations), for example through unconventional methods (such as hydraulic fracturing).

A lateral well typically comprises a substantially lateral segment. That is to say, a substantial segment (e.g. a majority, for example, greater than 50 %) of the total length of the lateral well (i.e. of the total length of the lateral well bore) extends substantially laterally underground. It will further be appreciated that the terms "lateral" and "horizontal" are, in the context of hydrocarbon exploration and extraction, considered to be equivalent in meaning. Accordingly, a lateral well may be referred to as a horizontal well and a lateral segment of a well may be referred to as a horizontal segment of a well. Nevertheless, a lateral well (and, equivalently, a lateral segment of a well) does not necessarily extend precisely horizontally relative to the ground surface but may also deviate from horizontal, for example by up to about 45°, as a lateral well is typically drilled along a path which follows the local geology and, in particular, local geological horizons. In addition, a lateral well typically also comprises an initial vertical segment. The length of the initial vertical segment of the lateral well is, however, typically short relative to the length of the lateral segment of the lateral well. For example, a lateral well may comprise: an initial vertical segment which extends substantially vertically downwards from the ground surface to a region of interest for hydrocarbon exploration and/or extraction (i.e. a landing zone); and a lateral segment which extends substantially laterally within the region of interest (e.g. following the local geological structure) from an end of the initial vertical segment to an opposing end of the lateral well.

The region may be sedimentary basin, e.g. a petroliferous (i.e. hydrocarbon-bearing sedimentary basin).

Each set of stratigraphic measurements may comprise: measurements of a stratigraphic parameter obtained from a plurality of rock samples (e.g. extracted) from the respective (e.g. lateral) well, each rock sample of the respective plurality of rock samples being associated with (e.g. derived from or extracted from) a different measurement location within the respective (e.g. lateral) well. Additionally, each set of stratigraphic measurements may comprise: corresponding measurements of a depth parameter for each measurement location in the respective (e.g. lateral) well.

A stratigraphic parameter may be a parameter indicative of a rock characteristic. A rock characteristic may be, for example, a rock age (e.g. a relative rock age or an absolute rock age), a rock composition (for example, an amount or concentration of a chemical species (e.g. an element, isotope or molecule) in the rock or an amount or concentration ratio of two or more chemical species in the rock), or a rock type (e.g. a type of rock or a type of mineral in the rock). Accordingly, the stratigraphic parameter may be a chronostratigraphic parameter indicative of relative rock age or absolute rock age. For example, the stratigraphic parameter may be a radiometric dating parameter or an estimated rock age (for example, obtained by a radiometric dating method such as rhenium-osmium (Re-Os) dating) or a stable isotope dating parameter such as a carbon isotope signature (for example, a parameter indicative of a ratio of $^{13}C$ to $^{12}C$ such as $\delta^{13}C$, $\delta^{13}C_{carb}$ or $\delta^{13}C_{org}$). Additionally or alternatively, the stratigraphic parameter may be a chemostratigraphic parameter indicative of rock composition, such as a parameter indicative of an amount of a chemical species (e.g. an element, isotope or molecule) or a ratio of two or more chemical species (e.g. a ratio of an amount of a first element to an amount of a second element). Additionally or alternatively, the stratigraphic parameter may be a lithostratigraphic parameter indicative of rock type (for example, a mineralogical type, a crystal structure, a mechanical property, etc.).

A depth parameter may be a parameter indicative of a subterranean depth of a measurement location. The depth parameter may be indicative of a subterranean depth of the measurement location relative to the local ground surface. For example, the depth parameter may be indicative of (e.g. may be) a vertical depth of the measurement location relative to the local ground surface. Alternatively, the depth parameter may be indicative of a depth of the measurement location relative to a different reference. For example, the depth parameter may be indicative of a depth of the measurement location relative to a local reference frame. For example, the depth parameter may be indicative of the depth of the measurement location relative to the local geological structure, for example relative to a local geological horizon. The local geological horizon may be defined by (e.g. correspond to) an interface (e.g. boundary) between layers of different rock type, age or composition at or adjacent the measurement location. For example, the measurement location may be located within a first layer of rock having a first type, age or composition and the local geological horizon may be defined by (e.g. correspond to) an interface (e.g. boundary) between the first layer of rock and a second layer of rock having a second type, age or composition different from the first type, age or composition.

The rock samples may be core samples. The skilled person will appreciate that a core sample is a cylindrical section of rock having standardised dimensions. For example, a core sample may be a cylindrical section of rock having a diameter of about 1 inch (1.00 inch = 2.54 cm). Plugs may be extracted from core samples for detailed analysis.

Alternatively, the rock samples may be cuttings samples. The skilled person will appreciate that a cuttings sample is a sample of drill cuttings obtained when a well is drilled. Drill cuttings typically comprise (e.g. consist of) relatively small, broken pieces of rock produced by drilling action and brought to the surface in drilling mud. Cuttings samples are commonly examined as part of mud logging (i.e., well logging) processes. Cuttings samples are not conventionally used for the detailed structural, chemical or radioisotope analysis required for stratigraphic modelling. However, the present inventors have developed methods which enable the use of cuttings samples in stratigraphic modelling.

For the avoidance of doubt, although each set of stratigraphic measurements is derived from (e.g. obtained from) a different (e.g. lateral) well in the region, the method of the first aspect does not necessarily include any stratigraphic measurement steps carried out directly on rock samples. For example, the method may comprise determining (e.g. identifying) stratigraphic correlations between two or more sets of stratigraphic measurements based on pre-existing stratigraphic measurement data. In some examples, the method is carried out (at least in part) by a computer (i.e. such that the method is a computer-implemented method) and the method comprises: the computer receiving the two or more sets of stratigraphic measurement (e.g. receiving the pre-existing stratigraphic measurement data); and the computer determining (e.g. identifying) the stratigraphic correlations between the two or more sets of stratigraphic measurements.

Nevertheless, the method may further comprise experimental measurement steps. For example, the method may further comprise: (i.e. prior to determining (e.g. identifying) stratigraphic correlations between the two or more sets of stratigraphic measurements) measuring values of the stratigraphic parameter, and corresponding values of the depth parameter, for each of the plurality of rock samples (e.g. cuttings samples). Accordingly, for each (e.g. lateral) well, the method may comprise: extracting the plurality of rock samples (e.g. cuttings samples) from the (e.g. lateral) well; and measuring values of the stratigraphic parameter, and corresponding values of the depth parameter, for each of the plurality of rock samples (e.g. cuttings samples) from the (e.g. lateral) well.

In embodiments in which the wells are lateral wells, the method may comprise (i.e. prior to determining (e.g. identifying) stratigraphic correlations between the two or more sets of stratigraphic measurements): correcting one or more of the sets of stratigraphic measurements to take into account a non-zero inclination of the respective lateral well relative to the local ground surface. As discussed hereinabove, a lateral well (or at least a lateral segment thereof) may be drilled along a path which follows (e.g., approximately) the local geological structure, e.g. so as to remain (e.g., approximately) within a region of interest for hydrocarbon exploration and/or extraction (i.e. a landing zone). It will be appreciated, however, that the local geological structure (e.g. a local geological horizon) is commonly inclined relative to the local ground surface. Accordingly, a lateral well (or at least a lateral segment thereof) will commonly have a non-zero inclination relative to the local ground surface. Therefore, the rock at two points spaced apart along the lateral segment may be of the same age, composition, type, etc. (and therefore correspond to the same layer of rock formed at the same time) and yet be identified at different depths relative to the local ground surface. Moreover, the rock found at the same depth relative to the local ground surface but spaced apart laterally along the length of the lateral segment may correspond to rock formed at different times and therefore having different properties. In addition, two different lateral wells (or at least lateral segments thereof) in the same region may be inclined at different angles with respect to the local ground surface. Accordingly, in order to achieve an meaningful comparison of data obtained from two or more different lateral wells in a region (i.e. such that stratigraphic correlations may be identified), it may be necessary to correct one or more (e.g. two or more, for example all) of the sets of stratigraphic measurements to take into account the (i.e. potentially different) non-zero inclination of each respective lateral well. Once this correction has been carried out, the sets of stratigraphic measurement may be expressed relative to the local geological structure such that the measurement data is comparable along each lateral well and across the region.

For example, it may be that the measured depth parameter for each measurement location is indicative of a subterranean depth of the said measurement location relative to the local ground surface. Correcting a set of stratigraphic measurements to take into account a non-zero inclination of the respective lateral well relative to the local ground surface may therefore comprise: determining the inclination of the lateral well relative to the local ground surface; and, for each measurement location in the lateral well, calculating a corrected depth parameter, indicative of a depth of the measurement location relative to a local geological horizon, taking into account the determined inclination.

Determining the inclination of the lateral well relative to the local ground surface may comprise determining the average inclination of a lateral segment of the lateral well relative to the ground surface. The average inclination of a lateral segment of the lateral well may be the inclination of an average straight line path for the lateral segment of the lateral well. The average straight line path may be a straight line path about which the lateral segment of the lateral well varies (e.g. fluctuates). Determining the average inclination of the lateral segment of the lateral well may therefore comprise: fitting a straight line to the path followed by the lateral segment of the lateral well; and determining the inclination of the straight line relative to the local ground surface. Fitting the straight line and determining the inclination may be achieved, for example, by plotting the lateral segment of the lateral well as a function of (a) vertical depth below the local ground level and (b) lateral distance travelled along the well bore (which may be approximated by a measurement of total distance (i.e. regardless of orientation) travelled along the well bore) and fitting a straight line to the plotted lateral segment. The lateral segment of the lateral well may be plotted on the basis of measurement data, for example obtained at a plurality of reference points (e.g. the measurement locations) along the lateral well path. The local ground surface may assumed to be flat and horizontal in this analysis.

The skilled person will appreciate that determining stratigraphic correlations between two or more sets of stratigraphic measurements comprises identifying measurements (i.e. data points) in each set of stratigraphic measurements which derive from the same layers of rock (i.e. from layers of rock which were deposited at the same time). By identifying measurements in each set of stratigraphic measurements which derive from the same layers of rock, a mathematical relationship (i.e. a correspondence relationship) between the two or more sets of stratigraphic measurements may be derived. The mathematical relationship (i.e. the correspondence relationship) may then be used to express (e.g. plot) measurement data from two or more of the wells (e.g. lateral wells) in the same reference frame.

The method may comprise determining stratigraphic correlations between the two or more sets of stratigraphic measurements by a graphic correlation method. The graphic correlation method may be carried out manually or may be automated, for example on a computer processor.

It may be that the two or more sets of stratigraphic measurements are two or more first sets of stratigraphic measurements each comprising measurements of a first stratigraphic parameter and the method further comprises determining stratigraphic correlations between the two or more first sets of stratigraphic measurements and two or more second sets of stratigraphic measurements. The two or more second sets of stratigraphic measurements may each comprise measurements of a second stratigraphic parameter different from the first stratigraphic parameter. The first and second stratigraphic parameters may be different types of parameter; for example, it may be that the first stratigraphic parameter is a chronostratigraphic parameter and the second stratigraphic parameter is a chemostratigraphic parameter. Alternatively, the first and second stratigraphic parameters may be of the same general type; for example, it may be that the first stratigraphic parameter is a first chronostratigraphic parameter (e.g., a stable isotope dating parameter such as $\delta^{13}C$) and that the second stratigraphic parameter is a second chronostratigraphic parameter (e.g., a radiometric dating parameter such as a Re-Os dating parameter). The two or more second sets of stratigraphic measurements may be obtained from the same lateral wells in the region as the two or more first sets of stratigraphic measurements. The skilled person will appreciate that measurements of any number of different stratigraphic parameters may be correlated in this way. By identifying correlations between measurements of *different* stratigraphic parameters from the same wells in the region, measurements of one stratigraphic parameter may effectively be used to fill in gaps (i.e. as a function of subterranean depth) between measurements of another stratigraphic parameter, and more (e.g., the most) constrained correlations may be identified.

The method may further comprise compiling a stratigraphic composite profile for the region based on the identified correlations. The stratigraphic composite profile may include two or more (e.g. all) of the two or more sets of measurements of stratigraphic measurements obtained for the region. The stratigraphic composite profile may be used to identify geological correlations across the region. Compiling the stratigraphic composite profile for the region may comprise smoothing the measurement data used to compile the stratigraphic composite profile, for example using splines. Smoothing the measurement data may enable geological trends to be identified more easily.

The method may further comprise constructing a sedimentary depositional model of the region based on the identified correlations. For example, the method may comprise determining (e.g. calculating) the rate at which sediment was deposited at different locations in the region, for example at different times in the past. Chronostratigraphic measurement data can be used to determine accumulation rates (which may be representative of both sedimentation rates and erosion events). A sedimentary depositional model can be used to predict the likely location of hydrocarbon deposits in the region, since the formation of different types of hydrocarbon is highly dependent on the sedimentary history of a region.

The method may further comprise targeting (e.g. selecting) a sub-region of the region for hydrocarbon exploration based on the identified correlations. For example, the method may comprise constructing the sedimentary depositional model of the region based on the identified correlations and targeting a sub-region of the region for hydrocarbon exploration using the sedimentary depositional model. The targeted sub-region may be a sub-region where the likely location of hydrocarbon deposits (for example, of a particular nature) is predicted.

The method may further comprise drilling a (e.g. lateral) well for hydrocarbon extraction in the targeted sub-region. The method may further comprise extracting hydrocarbons from the (e.g. lateral) well in the targeted sub-region.

As discussed hereinabove, it may be that one or more steps of the method are carried out by a computer. For example, it may be that the method comprises a computer determining the stratigraphic correlations between the two or more sets of stratigraphic measurements. The method may comprise the computer correcting one or more (e.g. all) of the sets of stratigraphic measurements to take into account a non-zero inclination of a (e.g. each respective) lateral well relative to the local ground surface. The method may comprise the computer determining the inclination of the respective lateral well relative to the local ground surface and calculating the corrected depth parameter. The method may comprise the computer carrying out the graphic correlation method. The method may comprise the computer compiling the stratigraphic composite profile for the region. The method may comprise the computer constructing the sedimentary depositional model of the region. The method may comprise the computer targeting the sub-region of the region for hydrocarbon exploration.

In a second aspect according to claim 12, a computer program comprises instructions which, when the program is executed by a computer, cause the computer to carry out one or more steps of the method according to the first aspect. In particular, it may be that the instructions, when the program is executed by the computer, cause the computer to carry out any combination of the steps of the method of the first aspect identified hereinabove as being carried out by (or being suitable for being carried out by) a computer.

In a third aspect according to claim 13, there is provided a (e.g. non-transitory) computer-readable medium storing the computer program (e.g. the instructions) according to the second aspect. The computer program (e.g. the instructions) may be stored as computer-executable program code.

In an example not forming part of the invention, there is provided a data carrier signal carrying (e.g. encoding) the computer program (e.g. the instructions) according to the second aspect. The computer program (e.g. the instructions) may be provided in the form of computer-executable program code.

In a fourth aspect according to claim 7, a method comprises determining (e.g. identifying) stratigraphic correlations between two or more sets of stratigraphic measurements, each set of stratigraphic measurements being obtained from different (e.g. non-overlapping) sections of the same lateral well in a region. It may be that the different sections of the same lateral well are different sections of a (i.e. the same) lateral segment of the same lateral well.

It may be that each set of stratigraphic measurements comprises: (a) measurements of a stratigraphic parameter obtained from a plurality of rock samples from the respective section of the lateral well, each rock sample of the respective plurality of rock samples being associated with (e.g. obtained or extracted from) a different measurement location within the respective section of the lateral well, and (b) corresponding measurements of a depth parameter for each measurement location in the respective section of the lateral well. The stratigraphic parameter may be a parameter indicative of a rock characteristic and the depth parameter may be a parameter indicative of a subterranean depth of a measurement location. The rock samples may be cuttings samples.

The method may further comprise, prior to determining stratigraphic correlations between the two or more sets of stratigraphic measurements: measuring values of the stratigraphic parameter, and corresponding values of the depth parameter, for each of the plurality of rock samples (e.g. cuttings samples).

The method may further comprise, prior to determining stratigraphic correlations between the two or more sets of stratigraphic measurements: correcting the two or more sets of stratigraphic measurements to take into account a non-zero inclination of the lateral well relative to the local ground surface.

The method may further comprise: determining stratigraphic correlations between the two or more sets of stratigraphic measurements by a graphic correlation method.

One or more steps of the method may be carried out by a computer. For example, it may be that the method comprises a computer determining the stratigraphic correlations between the two or more sets of stratigraphic measurements. The method may comprise the computer correcting the two or more sets of stratigraphic measurements to take into account a non-zero inclination of the lateral well relative to the local ground surface. The method may comprise the computer carrying out the graphic correlation method.

In a fifth aspect according to claim 12, a computer program comprises instructions which, when the program is executed by a computer, cause the computer to carry out one or more steps of the method according to the fourth aspect. In particular, it may be that the instructions, when the program is executed by the computer, cause the computer to carry out any combination of the steps of the method of the fifth aspect identified hereinabove as being carried out by (or being suitable for being carried out by) a computer.

In a sixth aspect according to claim 13, there is provided a (e.g. non-transitory) computer-readable medium storing the computer program (e.g. the instructions) according to the fifth aspect. The computer program (e.g. the instructions) may be stored as computer-executable program code.

In an eighth aspect, there is provided a data carrier signal carrying (e.g. encoding) the computer program (e.g. the instructions) according to the sixth aspect. The computer program (e.g. the instructions) may be provided in the form of computer-executable program code.

In an example not forming part of the invention, a method comprises measuring, by cavity ring down spectroscopic (CRDS) analysis, an isotopic signature of carbon in a cuttings sample obtained from a hydrocarbon well.

Cavity ring down spectroscopy (CRDS) involves measuring the wavelength-dependent absorption of laser light by a gaseous sample within a mirrored cavity. Absorption is typically measured following switching off of the laser, allowing measurements of absolute optical extinction. The path length for the laser light extinction is increased significantly by repeated reflection within the mirrored cavity. CRDS can be used to determine analyte concentrations down to the parts per trillion level. In particular, CRDS can be used to distinguish between different isotopes of the same element, and between molecules comprising different isotopes of the same element.

The isotopic signature of carbon may be a parameter indicative of the ratio of $^{13}C$ to $^{12}C$ in the cuttings sample. The parameter indicative of the ratio of $^{13}C$ to $^{12}C$ in the cuttings sample may be a parameter indicative of the ratio of a molecule comprising $^{13}C$ to a molecule comprising $^{12}C$. For example, the parameter indicative of the ratio of $^{13}C$ to $^{12}C$ in the cuttings sample may be a parameter indicative of the ratio of $^{13}CO_2$ to $^{12}CO_2$ in the cuttings sample.

The parameter indicative of the ratio of $^{13}C$ to $^{12}C$ in the cuttings sample may be $\delta^{13}C$, which is defined by

$$\delta^{13}\text{C} = \left( \frac{\left( \frac{^{13}C}{^{12}C} \right)_{\text{sample}}}{\left( \frac{^{13}C}{^{12}C} \right)_{\text{standard}}} - 1 \right) \times 1000 \text{ ‰} \qquad (1)$$

where $\left( \frac{^{13}C}{^{12}C} \right)_{\text{sample}}$ is the ratio of the amount of $^{13}C$ to $^{12}C$ in the sample analysed by the CRDS instrument,

$\left( \frac{^{13}C}{^{12}C} \right)_{\text{standard}}$ is the ratio of the $^{13}C$ to $^{12}C$ in a standard reference sample and $\delta^{13}C$ is measured in per mil (‰). The standard reference sample is the Cretaceous marine fossil, *Belemnitella americana,* from the Peedee Formation in South Carolina, which has a value of $^{13}C$:$^{12}C$ of 0.01118.

The method may comprise combusting the cuttings sample (or a portion thereof) in the CRDS instrument (i.e. for CRDS analysis).

The parameter indicative of the ratio of $^{13}C$ to $^{12}C$ in the cuttings sample may be $\delta^{13}C_{\text{org}}$. $\delta^{13}C_{\text{org}}$ is the value of $\delta^{13}C$ obtained when only considering the $^{13}C$ to $^{12}C$ ratio in a sample which derives from preserved organic carbon (in contrast to dissolved inorganic carbon).

The method may comprise cleaning the cuttings sample prior to CRDS analysis (i.e. prior to combusting the sample (or a portion thereof) in the CRDS instrument). For example, the method may comprise cleaning the cuttings sample to remove hydrocarbon residue (for example by solvent extraction) and/or inorganic carbonates (for example by dissolution in acid) prior to CRDS analysis (i.e. prior to combusting the sample (or a portion thereof) in the CRDS instrument).

Values output by the CRDS instrument may drift over a plurality of measurements. That is to say, the value output by the CRDS instrument for a sample at a first time may be different from the value output by the CRDS instrument for the same sample at a second time later than the first time, particularly when a plurality of other measurements have been made in the intervening time period. Accordingly, the method may comprise: prior to CRDS analysis, determining a drift in the output of the CRDS instrument; and (a) calibrating the CRDS instrument to compensate for the determined drift in the output or (b) compensating an output from the CRDS instrument for the cuttings sample based on the determined drift. For example, the method may comprise: prior to CRDS analysis, determining a drift in the output of the CRDS instrument based on measurements performed on a reference rock sample having a known isotopic signature of carbon; and (a) calibrating the CRDS instrument to compensate for the determined drift in the output or (b) compensating an output from the CRDS instrument for the cuttings sample based on the determined drift.

Values output by the CRDS instrument may depend on the mass of a sample being analysed. That is to say, the values output by the CRDS instrument for a first sample of a rock and a second sample of the same rock may be different due to the mass of the first sample being different from the mass of the second sample. Accordingly, the method may comprise: prior to CRDS analysis, determining a relationship between the output of the CRDS instrument and the mass of samples analysed; and (a) calibrating the CRDS instrument to compensate for the determined relationship or (b) compensating an output from the CRDS instrument for the cuttings sample based on the determined relationship. For example, the method may comprise: prior to CRDS analysis, determining a relationship between the output of the CRDS instrument and the mass of samples analysed based on plurality of measurements performed on a plurality of reference rock samples having the same known isotopic signature of carbon but differing in mass; and (a) calibrating the CRDS instrument to compensate for the determined relationship or (b) compensating an output from the CRDS instrument for the cuttings sample based on the determined relationship.

In an another aspect not forming part of the invention, a method comprises: for each hydrocarbon well (e.g. lateral hydrocarbon well) of two or more hydrocarbon wells (e.g. lateral hydrocarbon wells) in a region, measuring, by the method according to the ninth aspect, an isotopic signature of carbon for a plurality of cuttings samples from the respective hydrocarbon well (e.g. lateral hydrocarbon well), each cuttings sample being associated with a different measurement location within the hydrocarbon well (e.g. lateral hydrocarbon well), thereby generating two or more sets of carbon isotopic signature measurements, each set of carbon isotopic signature measurements being obtained from a different hydrocarbon well (e.g. lateral hydrocarbon well) in the region; and identifying correlations between the two or more sets of carbon isotopic signature measurements, for example by the method according to the first aspect.

In an another aspect not forming part of the invention, a method of estimating an age of a rock at a measurement location within a hydrocarbon well (e.g. a lateral hydrocarbon well) comprises performing rhenium-osmium (Re-Os) dating on a plurality of cuttings samples taken from the rock at the measurement location.

Re-Os dating may comprise measuring the $^{187}Os/^{188}Os$ and $^{187}Re/^{188}Os$ isotopic ratios of each of the cuttings samples. The $^{187}Os/^{188}Os$ and $^{187}Re/^{188}Os$ isotopic ratios may be measured using mass spectrometry, for example negative thermal ionization mass spectrometry.

The method may comprise: measuring $^{187}Os/^{188}Os$ and $^{187}Re/^{188}Os$ ratios for each cuttings sample; determining an isochron based on the measured $^{187}Os/^{188}Os$ and $^{187}Re/^{188}Os$ ratios; and estimating the age of the rock from the isochron (for example, based on the gradient of the isochron). The skilled person will appreciate that the isochron is a straight line fit to the experimentally measured $^{187}Os/^{188}Os$ and $^{187}Re/^{188}Os$ data (i.e. a straight line fit to the experimentally measured data points when plotted as a function of $^{187}Os/^{188}Os$ and $^{187}Re/^{188}Os$).

The $^{187}Os/^{188}Os$ and $^{187}Re/^{188}Os$ measurements are typically scattered. When the level of scattering is high, the inaccuracy in the predicted rock age is typically greater. Accordingly, the method may comprise grouping or selecting the $^{187}Os/^{188}Os$ and $^{187}Re/^{188}Os$ data in such a way as to reduce scattering. For example, the method may comprise: (a) responsive to determining that the scattering of the measured $^{187}Os/^{188}Os$ and $^{187}Re/^{188}Os$ values used to determine the isochron exceeds a predetermined threshold, for example by determining that the mean square weighted deviation (MSWD) of the measured $^{187}Os/^{188}Os$ and $^{187}Re/^{188}Os$ values exceeds a predetermined MSWD threshold, determining a new isochron based on the measured $^{187}Os/^{188}Os$ and $^{187}Re/^{188}Os$ ratios for a subset of the plurality of cuttings samples which are closer to one another in initial $^{187}Os/^{188}Os$ ratios; and (b) estimating the age of the rock from (e.g. based on the gradient of) the new isochron. The method may comprise: repeating steps (a) and (b) until the scattering of the measured $^{187}Os/^{188}Os$ and $^{187}Re/^{188}Os$ values used to determine the isochron is reduced to, or below, the predetermined (e.g. MSWD) threshold.

The method may comprise: determining the isochron based on measurements of the $^{187}Os/^{188}Os$ and $^{187}Re/^{188}Os$ ratios obtained from more than one pick from each cuttings sample. It will be appreciated that a "pick" is a sub-sample (i.e. a portion) of a cuttings sample.

In another aspect not forming part of the invention, a method comprises: for each hydrocarbon well (e.g. lateral hydrocarbon well) of two or more hydrocarbon wells (e.g. lateral hydrocarbon wells) in a region, determining, by the method according to the eleventh aspect, an age of rock at a plurality of different measurement locations within the hydrocarbon well (e.g. lateral hydrocarbon well), thereby generating two or more sets of rock age estimates, each set of rock age estimates being obtained from a different hydrocarbon well (e.g. lateral hydrocarbon well) in the region; and identifying correlations between the two or more sets of rock age estimates, for example by the method according to the first aspect.

**Figures**

Embodiments will now be described by way of example only, with reference to the Figures, in which:

**Figure 1** shows two schematic plots of measurements of a parameter, P, as a function of vertical depth, D, in wells A and B;

**Figure 2** illustrates schematically application of the graphic correlation method to the data sets of Figure 2;

**Figure 3** illustrates the path of an example lateral well bore, where the full path is shown in (a) and a magnified lateral segment of the path is shown in (b);

**Figure 4** illustrates schematically how the lateral segment of Figure 3 can be corrected to account for a non-zero inclination of the segment relative to the ground surface;

**Figure 5** illustrates the lateral segment of Figure 3 following correction according to Figure 4;

**Figure 6** illustrates schematically a measured parameter, P, plotted as a function of corrected depth TVD - $S_{cor}$;

**Figure 7** illustrates the parallel correlation of $\delta^{13}C_{carb}$ and $\delta^{13}C_{org}$ in the Paris Basin during the Toarcian Oceanic Anoxic Event;

**Figure 8** shows a plot of $\delta^{13}C_{org}$ values obtained for the same reference rock as a function of the mass of the rock sample, indicated by the volume of $^{12}CO_2$ generated on combustion of each sample;

**Figures 9 (a) and (b)**  illustrate eight lateral well paths in a petroliferous region;

**Figure 10** illustrates the SRS in the petroliferous region of Figures 9 (a) and (b);

**Figures 11 (a) to (g)** illustrate application of the graphic correlation method to each well in Figures 9 (a) and (b);

**Figure 12** illustrates a composite profile for the region which was obtained based on the graphic correlations found in Figures 9 (a) and (b);

**Figure 13** shows the composite profile of Figure 12 with smoothing using a spline algorithm;

Figures 14 (a) to (c) illustrate the $\delta^{13}C_{org}$ data obtained for each well in Figures 9 (a) and (b) alongside the smoothed composite profile for the region from Figure 13;

**Figure 15** illustrates an example lateral well path;

**Figure 16** illustrates division of the lateral well path of Figure 15 into sections for application of the graphic correlation method;

**Figure 17** illustrates XRF-measured Zr/CaO ratios obtained from cuttings samples extracted from a plurality of measurement locations in each section of Figure 16;

**Figures 18 (a) and (b)**  illustrate how correlations between the XRF data of Figure 17 can be obtained by graphic correlation to form the composite profile for the lateral well;

**Figure 19** illustrates chronostratigraphically-constrained spline-smoothed composite profiles for a plurality of lateral wells in a petroliferous region based on: $\delta^{13}C_{org}$; the ratio of total clay to total carbonate; the Zr/CaO ratio; and the Zr/Al$_2$O$_3$ ratio;

**Figures 20 (a) and (b)**  illustrate the correlations between Zr/CaO ratios measured for each individual lateral well contributing to the composite profile of Figure 19;

**Figure 21** illustrates an example isochron obtained from Re-Os dating measurements made on thirteen cuttings samples taken from a hydrocarbon well;

**Figure 22** shows the isochron produced from those cuttings samples of Figure 21 having an initial $^{187}Os/^{188}Os$ ratio of 0.55 ± 0.11;

**Figure 23** illustrates the isochron obtained using only "a" picks from the data of Figure 21;

**Figure 24** illustrates the isochron obtained using only "b" picks from the data of Figure 21; and

**Figure 25** shows a computer processor in communication with a computer-readable medium storing a computer program comprising computer-executable instructions.

## Detailed description

*Stratigraphy*

Stratigraphy is the branch of geology concerned with the study of stratified rocks and deals with the correlation of rock strata from different localities. Correlations between rock strata may be studied on the basis of fossilised flora and/or fauna (biostratigraphy), rock units or type (lithostratigraphy), elemental and/or isotopic composition (chemostratigraphy), or geologic time units or intervals (chronostratigraphy). A sequence stratigraphic model of an area identifies the order or sequence in which depositionally related rock units in the area were laid down. Sequence stratigraphic models, therefore, can be based on and incorporate lithostratigraphic, biostratigraphic, chemostratigraphic and chronostratigraphic data. In common usage, the term "stratigraphy" also refers to the relative spatial and temporal arrangement of rock strata in a given locality.

A detailed sequence stratigraphic understanding of an area is an important aid to effective and efficient hydrocarbon exploration. This is because a stratigraphic sequence at a given location in a sedimentary basin provides a history of the deposition of sediment at that location. By correlating stratigraphic sequences recorded at a plurality of locations across the basin, a model of the sedimentary history for the entire basin may be developed. Such a model enables the likely location of hydrocarbon deposits in the basin to be identified, given that the vast majority of subterranean hydrocarbon deposits were formed from fossilised organisms buried under marine or lacustrine sedimentary layers. An accurate stratigraphic model of an area is particularly useful when considering extraction of hydrocarbons from unconventional sources (such as oil shales or tight rock formations) through unconventional methods (such as the use of lateral wells and/or hydraulic fracturing). For example, international patent application WO2016011384 describes how stratigraphic methods can be combined with geochemical analysis to develop a predictive depositional model of a sedimentary basin for determining a location and areal extent of total organic carbon within the basin rock formation.

Other stratigraphic correlation methods are described in US 2016/018556 A1, WO 2015/070022 A1, OU CHENGHUA ET AL: "Fine reservoir structure modeling based upon 3D visualized stratigraphic correlation between horizontal wells: methodology and its application",JOURNAL OF GEOPHYSICS AND ENGINEERING, INSTITUTE OF PHYSICS PUBLISHING, BRISTOL, GB, vol. 14, no. 6, 22 November 2017, Mohsen Abdul ET AL: "Horizontal Well Correlation Using Real Time Data and Log Prediction in Geosteering Complex Reservoirs of Saudi Arabia",, 13 March 2013 and EL GEZEERY T ET AL: "Innovative Geosteering Technology Utilized in Drilling Smart Multi-lateral Wells, Kuwait",OFFSHORE TECHNOLOGY CONFERENCE, 6 September 2013.

At a fundamental level, a stratigraphic model of an area is a model of the variation of rock strata below ground across that area. For example, a stratigraphic model may identify the depth at which particular rock types (i.e., a lithostratigraphic model) or particular elements (i.e., a chemostratigraphic model) are located. As mentioned hereinabove, a stratigraphic model typically identifies correlations between rock strata at different localities within the area. The sequence of rock strata observed at the different locations may be different, even within the same sedimentary basin, due to, for example, variations in depositional environments across the basin as a function of time (e.g., due to changing sea, lake and river levels) or due to localised erosion at particular time periods.

Analysing the layering of a single stratigraphic column enables *relative* dating of the strata within that column. Moreover, correlating one stratigraphic column with another can sometimes enable rock units of the same age to be identified in different columns, thereby enabling *relative* dating of the strata across an area (although it must be borne in mind that rocks having the same lithology, for example, did not necessarily form at the same time). A chronostratigraphic model, which enables *absolute* dating of the rock strata in a column and across an area, can also be developed by studying correlations in, for example, fossilised flora and fauna, magnetic properties of the rock, radioisotope concentrations, etc. Chronostratigraphic analysis, in particular, enables calculation of historic sedimentary deposition rates, which are known to have had a strong influence on hydrocarbon formation. Stratigraphic models can therefore assist an explorer in identifying the location of suitable hydrocarbon-bearing rock layers and in directing well drilling operations appropriately.

When extracting hydrocarbons from unconventional sources such as oils shales or tight rock formations, lateral wells (also known as horizontal wells) are often required. While the general "landing zone" for lateral well drilling may be relatively wide (e.g., 10 to 20 metres thick), the optimum region for hydrocarbon extraction within the landing zone may be relatively narrow (e.g., less than 1 metre thick). A region may be considered an optimum region for hydrocarbon extraction due to the quantity or concentration of particular hydrocarbons in that region and/or the mechanical properties of the rock in that region which make it particularly suitable for hydrocarbon extraction (e.g., by hydraulic fracturing). The more detailed a stratigraphic model of an area is, the more accurately explorers can target the optimum region for hydrocarbon extraction.

*Graphic correlation*

Graphic correlation is a method for generating a stratigraphic model of an area using experimental data obtained from samples taken from multiple wells spaced apart from one another across the area. While the graphic correlation method was first developed in the field of biostratigraphy when investigating the fossil record, it has since also been used to correlate non-paleontological data between wells. Graphic correlation has, in fact, become a standard method in the field and is described in detail in, for example, MANN, Keith Olin. Graphic Correlation. Tulsa, Oklahoma, USA: SEPM Society for Sedimentary Geology, 1995, ISBN 9781565760233. As discussed in international patent application WO2016011384, graphic correlation can be used: to verify experimental data obtained through measurements of a parameter from a single well by correlating that data with a known reference; to verify experimental data obtained through measurements of different parameters from a single well by correlating those measurements with one another; and/or to identify correlations between measurements obtained from different wells.

While the skilled person is well aware of the graphic correlation method as applied to data obtained from samples from the vertical wells commonly used in conventional hydrocarbon exploration, the present inventors have extended the analysis method in order to examine correlations between the lateral wells used in unconventional hydrocarbon exploration. The present inventors have also developed improved experimental techniques which enable the identification of correlations at a finer scale than is commonly achievable, particularly when considering chronostratigraphic correlations. By way of background to the discussion of the inventors' new developments in the field, a brief explanation of the graphic correlation method as commonly applied to vertical wells (in order to identify correlations between those wells) now follows.

*Graphic correlation between vertical wells*

The first stage in the graphic correlation method involves the collection of data sets, each data set derived from a different vertical well in a given area. The data set for each well contains a series of measurements of a particular parameter, $P$, as a function of vertical depth, $D$, in the well. Any suitable parameter, $P$, may be studied. For example, the parameter, $P$, may be indicative of a rock type, an amount of an element or isotope present in the rock, or the presence of particular fossilised flora or fauna, etc. The parameter, $P$, is commonly measured on the basis of core samples extracted from each well, a core sample being a cylindrical section of the rock having standardised dimensions.

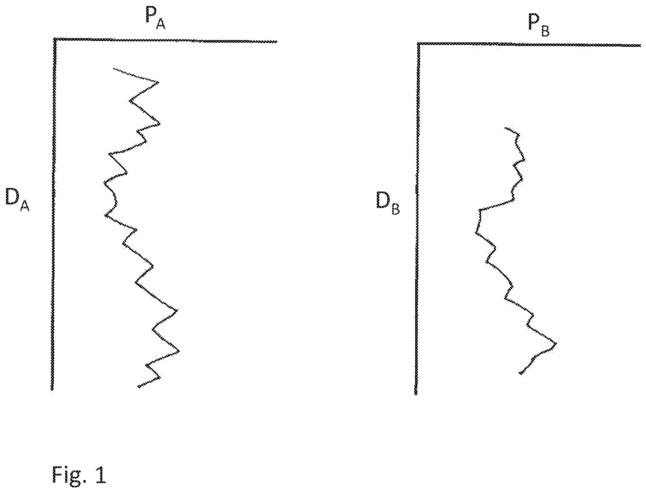

The second stage in the graphic correlation method involves identifying a Stratigraphic Reference Section (SRS). In particular, the well data set having the largest stratigraphic coverage (i.e., including values of $P$ across the largest vertical depth) is selected as the SRS against which all other data sets will be correlated and referenced. For example, data sets of the parameter, $P$, as a function of vertical depth, $D$, may be collected for two wells labelled A and B, as illustrated schematically in Figure 1. The data set for well A has the largest stratigraphic coverage (i.e., spans the largest depth interval) and is therefore selected as the SRS.

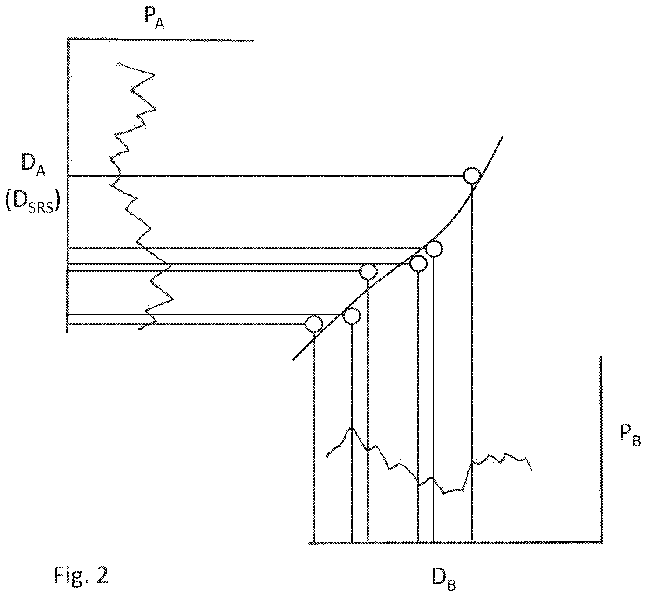

The third stage is to carry out the graphic correlation between the data sets. This stage is illustrated in Figure 2. The data set obtained from well B is plotted against the SRS (i.e., well A). In particular, the SRS data set is plotted in the upper left corner of Figure 2, while the data set for well B is plotted in the lower right corner. The axes are arranged such that $D$ values are plotted on the vertical axis for the SRS but on the horizontal axis for well B. Data points in the data sets for the SRS and well B which are known to be correlated with one another are identified. These specific correlations (known as 'picks') are identified by comparing the SRS and well B data sets to data relating to known regional or global geological or palaeontological events (for example, known from existing chemostratigraphic, biostratigraphic or seismic data). For example, regional correlation picks in individual wells may be based on interpretation of seismic data, for example by identifying and mapping seismic horizons (i.e., seismic reflectors which reflect a change in rock properties (e.g., seismic velocity and/or density) across a boundary between two layers of rock) from well to well across a region. Regional correlation picks may also be identified based on petrophysical gamma ray log data, assuming that there is a consistent basin-wide geological control over the gamma ray profile.

Horizontal lines are drawn through the correlated points in the SRS data set and vertical lines are drawn through the corresponding correlated points in the well B data set. The points of intersection of the vertical and horizontal lines are marked in Figure 2 by open circles. Once a plurality of such known correlation points are identified, a best-fit line may be drawn, as shown in Figure 2, through the points of intersection. This best-fit line is referred to as the graphic correlation line.

If the correlation between the wells were exactly 1:1 (e.g., such that the thickness and depth of the corresponding strata in both wells were the same), the graphic correlation line would be straight. However, generally speaking, the thickness and position of the strata varies from well to well across the region (due to the specific geologic history of each stratigraphic column) and, therefore, the graphic correlation line is not straight. Nevertheless, an equation for the best-fit graphic correlation line may be determined in terms of $D_A$ (i.e., $D_{SRS}$) and $D_B$. equation of the graphic correlation line can then be taken as an expression defining the correlation between the two data sets. Therefore, depth values, $D_B$, from the well B data set can be converted into equivalent $D_{SRS}$ values such that that the $P_B$ data from well B can be plotted together with the $P_A$ data as a function of $D_{SRS}$ (i.e., against the same $D_{SRS}$ axis). Once this transformation has been performed, samples from well A and well B which are associated with the same value of $D_{SRS}$ are understood to come from rock of the same age. Accordingly, the $D_{SRS}$ axis can be thought of as a time axis, regardless of the nature of the parameter $P$ used in the construction of the graphic correlation (i.e., even if the parameter $P$ is not itself a measure of rock age).

The graphic correlation method is typically applied to data sets acquired from many different vertical wells in a region. Once the correlation between each well data set and the SRS has been obtained, and the depth values for each well data set have been transformed into equivalent $D_{SRS}$ values, the available $P$ values for every well in the region can be plotted together against the same $D_{SRS}$ axis. This plot is referred to as the 'composite profile' for the area, against which all wells are stratigraphically correlated. The composite profile data is commonly smoothed, for example by defining a smoothed line through all of the data points using a split fit of a moving average.

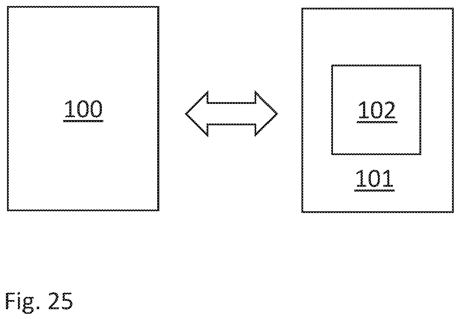

While the graphic correlation method has been illustrated here graphically, the skilled person will appreciate that this analysis is suited to automation and implementation in computer software (for example, computer software 102 stored on a computer-readable medium 101, for execution by a computer processor 100, as shown in Figure 25). With the aid of a computer processor, a composite profile correlating data obtained from many different wells can be obtained rapidly.

*Graphic correlation between lateral wells*

The inventors have adapted the known graphic correlation method in order to analyse correlations between the lateral wells used in unconventional hydrocarbon exploration.

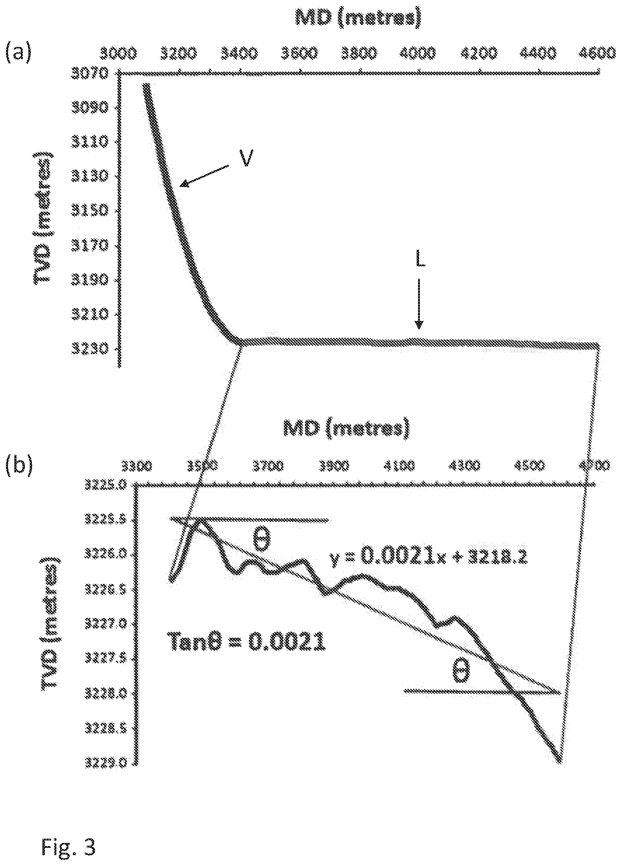

Figure 3 illustrates the path of an example lateral well bore. The entire path of the well bore is shown in Figure 3 (a), while a lateral segment of the path is magnified in Figure 3 (b). The path is illustrated by way of a plot of measured depth (MD) versus total vertical depth (TVD). The TVD is the total vertical depth of any point along the well bore path relative to the ground surface. The MD is the total distance along the well bore path between a given point on the path and the start of the well bore.

As can be seen in Figure 3 (a), the well bore path contains two principal segments: a "vertical" segment (labelled 'V') in which the TVD appears to increase continuously as the MD increases; and a "lateral" segment (labelled 'L') in which the TVD appears to remain approximately constant as the MD increases. In the lateral segment of the well bore path, since the rate of change of TVD with respect to MD is low, MD can be considered to be an approximate measure of the horizontal distance travelled by the well bore.

However, as can be seen in magnified Figure 3 (b), TVD is *not* constant along the lateral segment of the well bore path. Instead, the TVD is seen to fluctuate along the lateral segment. Moreover, the average path in the lateral segment is often not horizontal. This is because the well bore's trajectory tends to follow the local geologic structure (identified from, e.g., seismic data) and the landing zone does not necessary extend horizontally in all directions. In the example shown in Figure 3 (b), the depth of the well bore in the lateral segment fluctuates about an average path which slopes downwards away from the vertical segment.

Measurements of a parameter, P, (for example relating to the rock type, amount of an element or isotope present in the rock) may be made at different points along the lateral segment of the well bore. It would be desirable to include this data in a graphic correlation of the area. It would also be desirable to correlated measurements made along a single lateral path. In particular, due to the small changes in the depth of the well bore along the lateral segment, measurements taken at several points along the lateral segment could be used to construct finely detailed well correlations over a relatively narrow depth range, which in turn could be used to assist in accurately targeting the best rock within the landing zone for hydrocarbon extraction. However, the standard graphic correlation method described in the preceding section is not directly applicable to the data obtained from lateral wells because the lateral segment of each well bore path is not horizontal, nor is the average path of the lateral segment the same in each well (since each lateral well follows the local geologic structure which typically varies between well locations). That is to say, measurements made at the same TVD within a given lateral well (for example, measurements made at two different locations along the well path which are spaced apart in terms of MD but which have the same TVD) are not directly comparable with one another because they may relate to different strata (e.g., rock of different age, lithology, etc.).

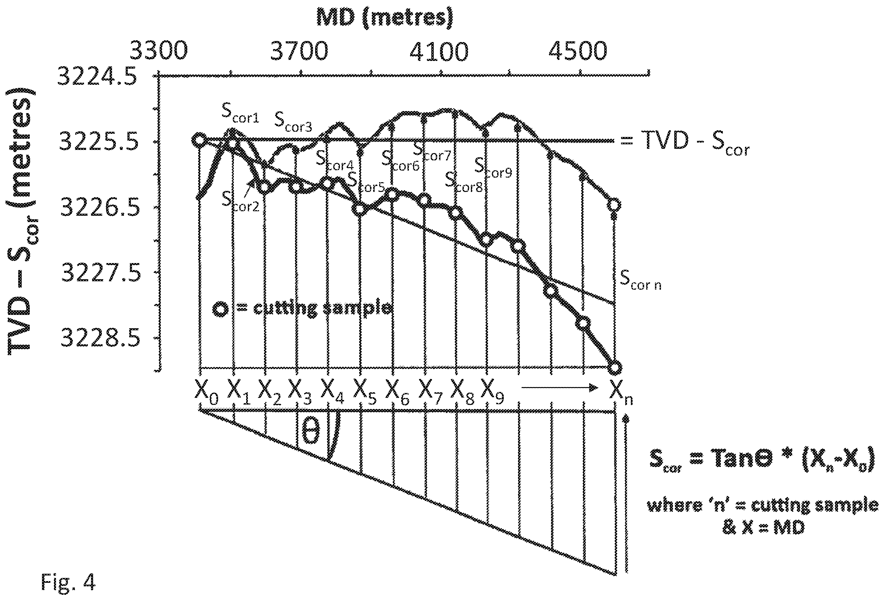

Nevertheless, the present inventors have developed a methodology which enables the graphic correlation of data obtained from lateral well bores. In particular, assuming that each lateral segment approximately follows its own local geology, a correction can be applied to the measurement data obtained from each well to bring the data into the same reference frame and enable comparison both along each lateral segment and between different sites. This process is illustrated in Figure 3 (b) and Figure 4.

First, the average inclination of the borehole along the lateral segment of a well is determined by calculating a best-fit straight line through the lateral segment in terms of TVD and MD, as shown in Figure 3 (b) (where *y* corresponds to TVD and *x* corresponds to MD). Then, as shown in Figure 4, the vertical depth of each point along the lateral segment (illustrated by open circles in Figure 4) is shifted vertically upwards by an amount, $S_{cor}$, determined according to

$$S_{cor} = \tan\theta\,(X_n - X_0) \qquad\qquad (2)$$

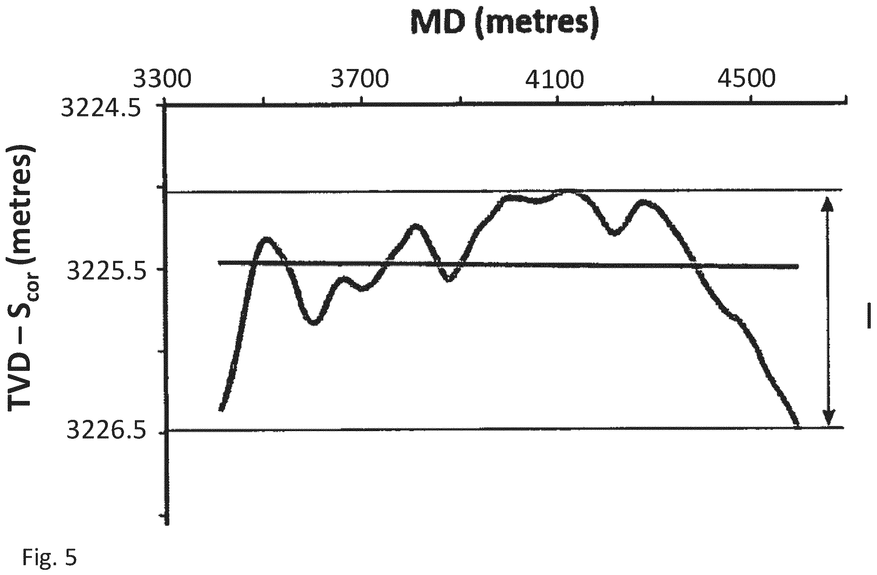

where $\theta$ is the angle of inclination of the best-fit straight line and $(X_n - X_0)$ is the distance (in MD) between a point *n* along the lateral segment and the beginning of the lateral segment (i.e. where the vertical and lateral segments meet). The result is a vertically corrected lateral bore path which fluctuates around a horizontal line when plotted against TVD - $S_{cor}$, as illustrated in Figure 5. Essentially, the well path is plotted relative to the local geological structure such that horizontal lines in Figure 5 intersect the well path at locations where the rock is of the same geological age.

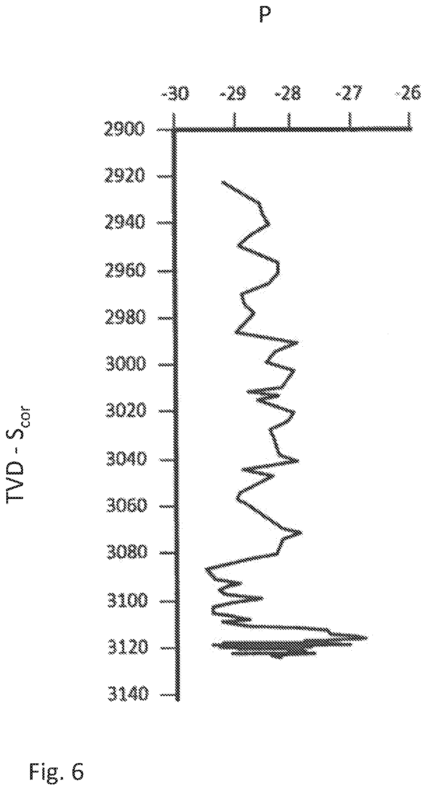

Once an equivalent correction has been applied to each data set from each lateral well in an area, the graphic correlation method can be used to study correlations in P along and between the lateral segments of each of the wells. For example, in order to identify correlations between a plurality of horizontal wells, as in the standard graphic correlation method, the well covering the largest stratigraphic interval (i.e., largest corrected depth (TVD - $S_{cor}$) interval) is selected as the SRS. The stratigraphic interval, *I*, of a particular lateral well is illustrated in Figure 5. For each well data set, including the SRS, the measured *P* values are plotted as a function of corrected depth (TVD - $S_{cor}$), for example as shown in Figure 6. The graphic correlation method as described in the preceding section may then be applied as usual to construct a composite profile from the plurality of depth-corrected data sets. The composite profile data may be smoothed, for example using a spline function, as described hereinabove.

It will be appreciated that, although the depth correction method for lateral well bores been illustrated here graphically, this analysis is suited to automation and implementation in computer software (for example, computer software 102 stored on a computer-readable medium 101, for execution by a computer processor 100, as shown in Figure 25), as with the standard graphic correlation method.

*Fine-scale chronostratigraphy*

As discussed hereinabove, application of the graphic correlation method to lateral segments of lateral wells, which tend to vary in depth along the lateral well path, can be used to identify correlations over relatively narrow depth intervals. A detailed understanding of correlations in a relatively narrow depth interval is particularly important when considering unconventional hydrocarbon exploration, where the optimum path for the lateral well bore should be targeted precisely. However, it is both difficult and expensive to obtain sufficient data (as required for the analysis of fine correlations within a relatively narrow depth interval) from lateral wells when the analysis is based on measurements performed on core samples, as is the standard in the art. More generally, even when considering data obtained from conventional vertical wells, it can be difficult and expensive to obtain core samples for analysis.

One alternative to the use of core samples is to carry out the required measurements on cuttings samples. Cuttings samples are samples of the drill cuttings obtained when a well is drilled; drill cuttings are typically small, broken pieces of rock produced by the drilling action and brought to the surface in the drilling mud. Cuttings are typically examined as part of mud logging (i.e., well logging) analysis, which includes observation of the cuttings, microscopic examination and basic chemical analysis. It is, however, generally understood that cuttings samples are not suitable for the detailed structural, chemical or radioisotope analysis required for stratigraphic modelling.

Nevertheless, the present inventors have developed a method for isotopic dating of cuttings samples which generates data suitable for use in chronostratigraphy. Because cuttings samples are plentiful, rock may be sampled at many locations along a well bore without significant increase in cost or complexity. Very finely detailed chronostratigraphic correlations may therefore be obtained.

The new method is based on Cavity Ring Down Spectroscopy (CRDS) of carbon isotopes.

In particular, it is known that the ratio of $^{13}C$ and $^{12}C$ isotopes in a rock sample can be used to establish an age for the sample. A parameter, $\delta^{13}C$ (in per mil (‰)) is defined by:

$$\delta^{13}\text{C} = \left( \frac{\left(\frac{^{13}C}{^{12}C}\right)_{\text{sample}}}{\left(\frac{^{13}C}{^{12}C}\right)_{\text{standard}}} - 1 \right) \times 1000 \ \text{‰} \qquad (3)$$

The standard ratio of $^{13}\text{C}$ and $^{12}\text{C}$ is based on an established reference material, the Pee Dee Belemnite Cretaceous marine fossil *Belemnitella Americana,* which has a ratio of 0.0112372. Based on this standard, most natural materials have a negative $\delta^{13}\text{C}$.

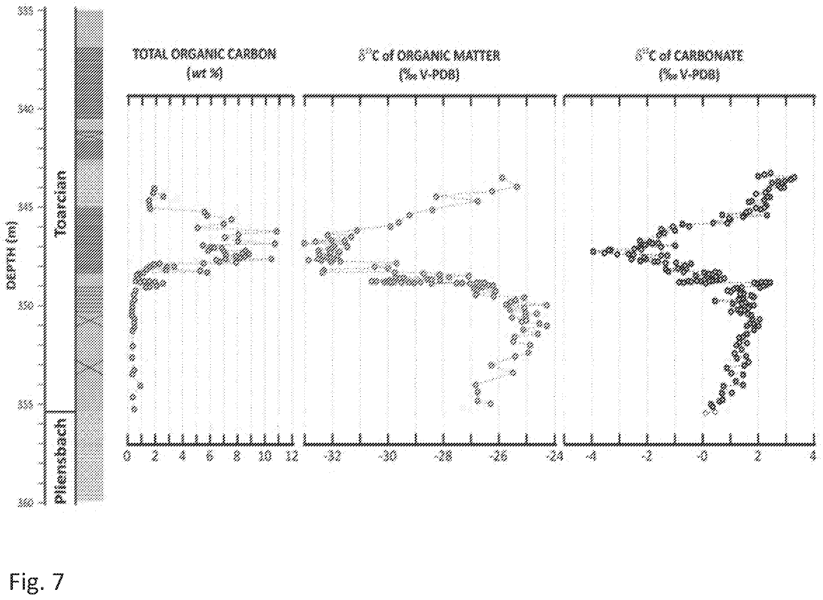

$\delta^{13}\text{C}$ can be used to date geological samples because the proportions of C isotopes incorporated in biogenic material has changed through time in response to fluctuating palaeoenvironmental and geological conditions. The primary biogenic signal, however, can be masked by diagenetic alteration and it can be necessary to disentangle the primary biogenic signal from secondary diagenetic effects. This can be achieved by splitting $\delta^{13}\text{C}$ into $\delta^{13}\text{C}_{\text{carb}}$, which reflects changes in dissolved inorganic carbon (DIC), and $\delta^{13}\text{C}_{\text{org}}$, which reflects changes in preserved organic carbon (OC), both of which are linked to atmospheric carbon dioxide concentrations. For example, Figure 7 illustrates the strong parallel correlation of $\delta^{13}\text{C}_{\text{carb}}$ and $\delta^{13}\text{C}_{\text{org}}$ in the Paris Basin during the Toarcian Oceanic Anoxic Event.

Since measurements of $\delta^{13}\text{C}_{\text{org}}$ tend to correlate with $\delta^{13}\text{C}_{\text{carb}}$, and $\delta^{13}\text{C}$ more generally, $\delta^{13}\text{C}_{\text{org}}$ can be used to date samples containing organic matter. In particular, measurements of $\delta^{13}\text{C}_{\text{org}}$ by CRDS can be used to date samples of organic kerogen (i.e., the solid, insoluble organic matter found in sedimentary rocks such as oil shales).

Before CRDS is carried out to measure $\delta^{13}\text{C}_{\text{org}}$ from cuttings samples, samples must be prepared to remove hydrocarbon residue, both naturally occurring and introduced in Oils based Mud (OBM) systems, and inorganic carbonates, leaving behind the organic kerogen for analysis. Hydrocarbons can be removed by a solvent extraction method. For example, the cuttings samples may be powdered and the hydrocarbons may be removed by rinsing the powdered samples in toluene repeatedly until the toluene remains clear during the rinse. Carbonates can be removed by dissolution in acid. For example, carbonates are removed using 10% hydrochloric acid (i.e., HCl). In such a process, the hydrochloric acid may be introduced to the toluene-rinsed, powdered sample and left in a 40 °C water bath for 24 hours to ensure that all carbonate has been removed.

Following sample preparation, the $\delta^{13}\text{C}_{\text{org}}$ value is measured using a Picarro CRDS instrument (available from Picarro, Inc., Santa Clara, USA) as is standard in the art. The sample is combusted to produce $CO_2$ within a mirrored cavity through which an infrared laser is shone. By measuring the absorption of laser light at different wavelengths, the CRDS instrument is able to determine accurately the relative amounts of $^{12}CO_2$ and $^{13}CO_2$ in the cavity. The value of $\delta^{13}\text{C}_{\text{org}}$ obtained can then be compared to standard references in order to date the sample.

While CRDS enables detection of gaseous species down to the parts per billion level, and sometimes parts per trillion level, the inventors have found that the measurements obtained from the CRDS instrument tend to drift as multiple samples are tested. Accordingly, it is necessary to recalibrate the instrument periodically or to correct the measurements for drift. A recalibration or correction every five measurements has been found to be sufficient. In practice, correction is achieved by measuring the $\delta^{13}\text{C}_{\text{org}}$ value for a reference sample having a known $^{12}\text{C}{:}^{13}\text{C}$ ratio, calculating the error $X$ in the $\delta^{13}\text{C}_{\text{org}}$ value, and then adjusting the measured $\delta^{13}\text{C}_{\text{org}}$ values for the next five samples by the amount $X$ (i.e., by adding or subtracting $X$ to or from the measured value, dependent on the direction of the drift), following which the reference sample is retested and the error recalculated.

The inventors have also found that the output from the CRDS instrument is sensitive to changes in the volume of $CO_2$ produced during kerogen combustion. The volume of $CO_2$ produced is related to the amount of organic matter (i.e., carbon) in a sample. It is therefore necessary to correct the output from the CRDS instrument based on the weight of organic matter in a sample. However, the total weight of organic matter in a sample is generally unknown and its determination is complex and time-consuming. The inventors, however, have developed a simplified method for correcting the output from the CRDS instrument based on the total weight of the sample to be analysed.

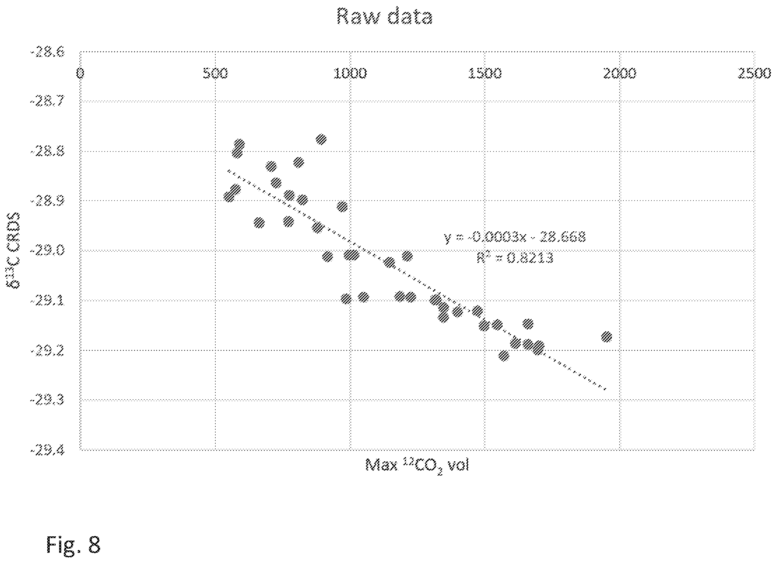

$\delta^{13}\text{C}_{\text{org}}$ values are measured by CRDS for a series of reference rock samples (i.e., taken from a rock having a known $\delta^{13}\text{C}_{\text{org}}$ value) having different weights, for example increasing in weight by 0.1 mg increments across a weight range of interest. The rock samples are obtained from the same rock and so should produce the same $\delta^{13}\text{C}_{\text{org}}$ values. The results of an example series of measurements are shown in Figure 8, plotted as a function of the volume of $^{12}CO_2$ produced (which is measured by the CRDS device and which correlates with the weight of carbon in the samples). As can be seen in Figure 8, there is a negative trend between the measured $\delta^{13}\text{C}_{\text{org}}$ value and the volume of $^{12}CO_2$ output by the CRDS device. A best-fit straight line through the data is obtained and the equation of the line is determined.

The equation of the best-fit line can be used to correct the output from the CRDS instrument for a given sample weight. That is to say, the $\delta^{13}\text{C}_{\text{org}}$ value for an unknown sample is measured and the volume of $^{12}CO_2$ produced is also obtained from the CRDS instrument. The equation of the best-fit trend line is then used to correct the $\delta^{13}\text{C}_{\text{org}}$ value based

on the known volume of $^{12}CO_2$ produced, assuming that the gradient of the trend line is the same for the rock sample being measured as it is for the reference sample. In particular, the trend line has a general equation given by

$$\delta^{13}C_{org} = mV_{CO_2} + c \qquad (4)$$

where $V_{CO_2}$ is the volume of $^{12}CO_2$, $m$ is the gradient of the trend line and c is the $\delta^{13}C_{org}$ intercept, where $m$ and c are determined from the plotted data for the reference rock samples. It is also known that the reference value of $\delta^{13}C_{org}$ is obtained from the CRDS instrument when $V_{CO_2}$ is equal to the reference volume, $V_R$. Assuming that the gradient of the trend line is constant, the $\delta^{13}C_{org}$ value output by the CRDS instrument for an unknown sample ($\delta^{13}C_M$) and the corresponding volume of $^{12}CO_2$ produced ($V_M$) are related to one another according to

$$\delta^{13}C_M = mV_M + d \qquad (5)$$

The corresponding corrected (i.e. 'true') value of $\delta^{13}C_{org}$ for the unknown sample ($\delta^{13}C_T$), at the volume $V_R$, is given by

$$\delta^{13}C_T = mV_R + d \qquad (6)$$

By subtracting Equation (5) from Equation (6) and rearranging, an expression for the corrected $\delta^{13}C_{org}$ value can be obtained as

$$\delta^{13}C_T = \delta^{13}C_M + m(V_R - V_M) \qquad (7)$$

That is to say, the corrected value of $\delta^{13}C_{org}$ is obtained by applying a correction of $m(V_R - V_M)$ to the measurement output by the CRDS instrument.

The correction parameters must be obtained for each particular CRDS instrument and should also be retested periodically for the same instrument, for example every six months, and following any changes to (such as maintenance of) the CRDS instrument.

The inventors have found that use of the CRDS method enables a large number of cuttings samples to be analysed quickly and at low cost. Accordingly, $\delta^{13}C_{org}$ measurements can be taken at many locations along a well bore (whether vertical or lateral), enabling very finely detailed chronostratigraphic models to be developed.

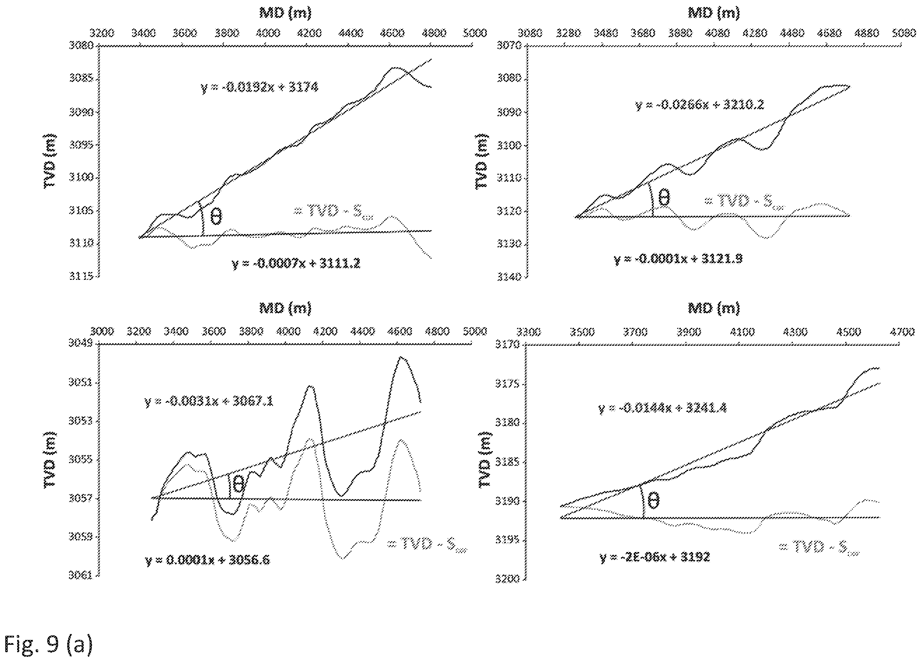

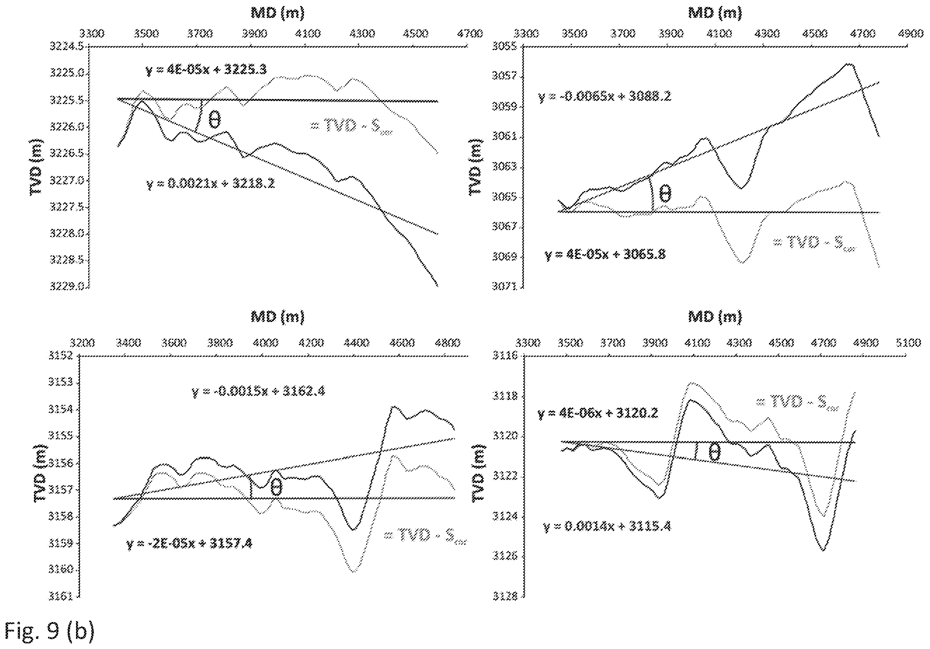

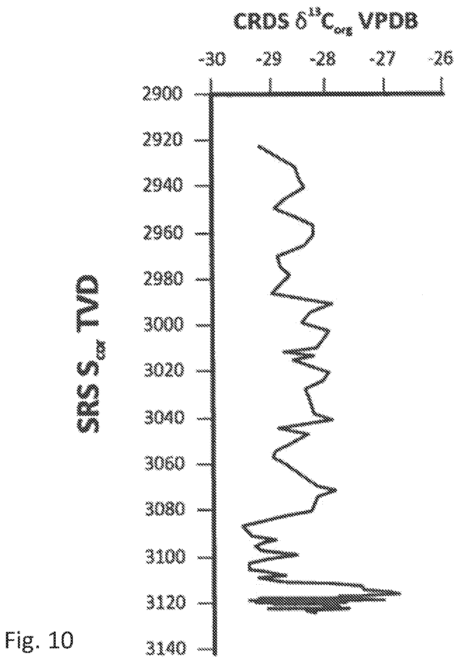

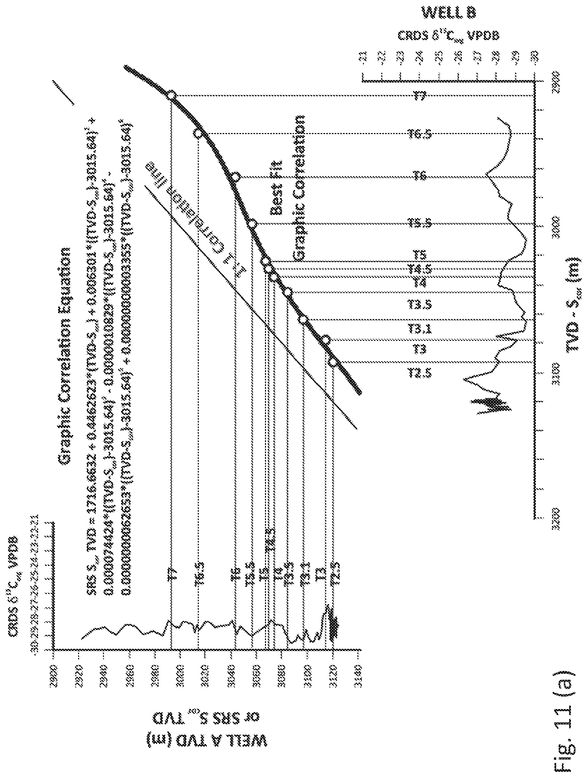

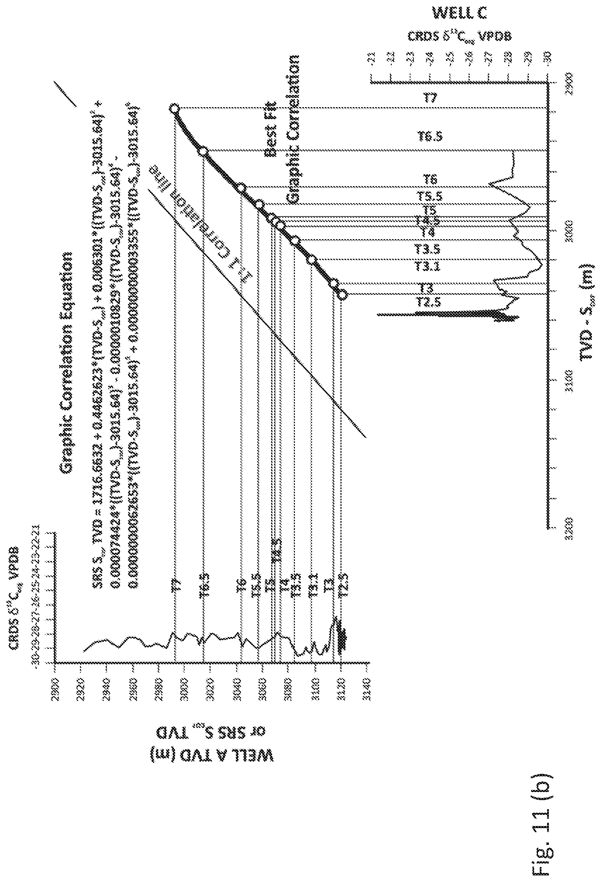

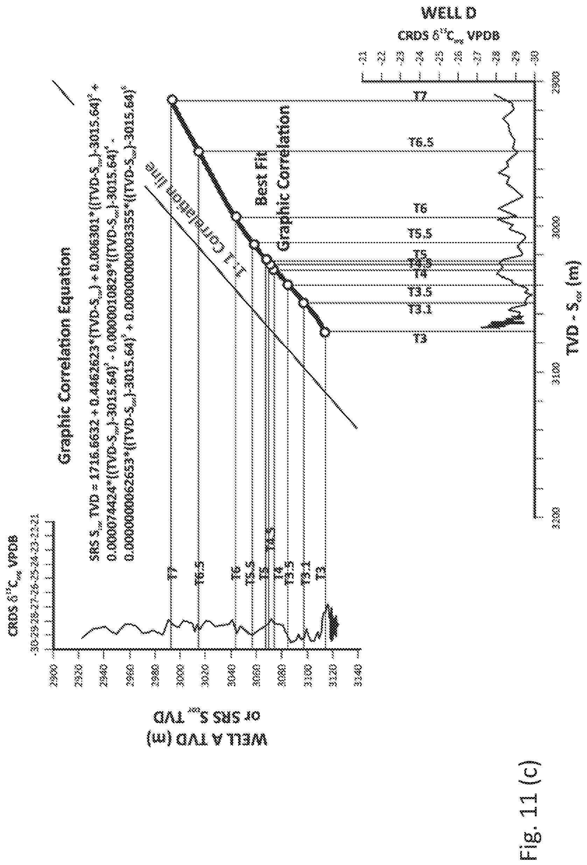

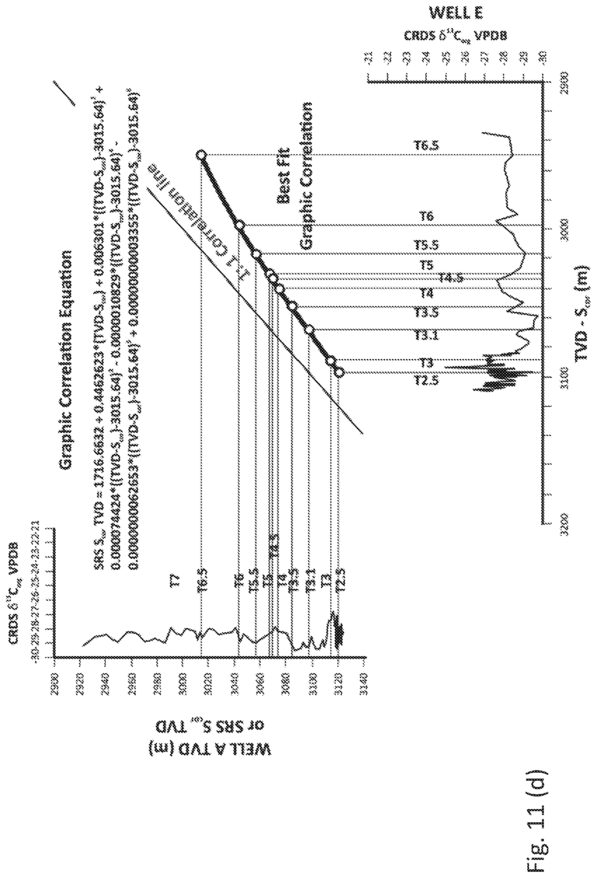

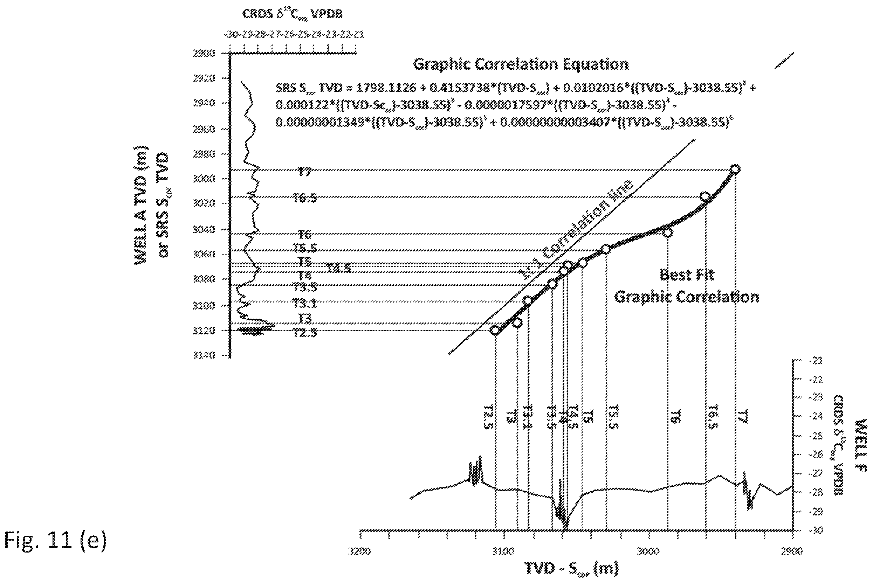

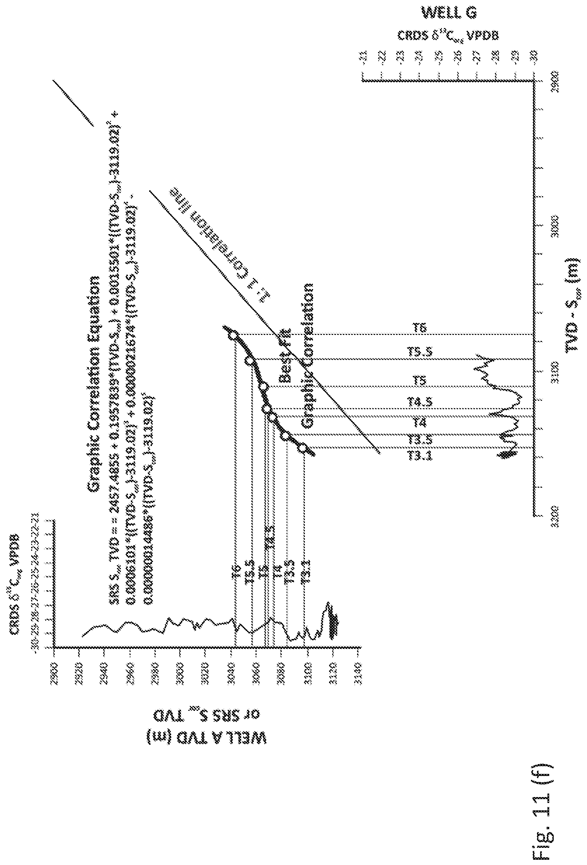

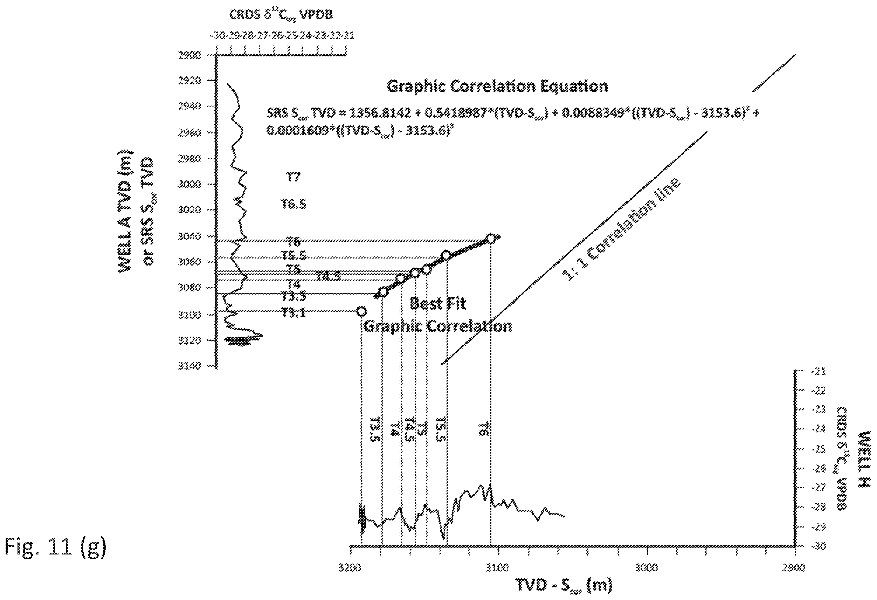

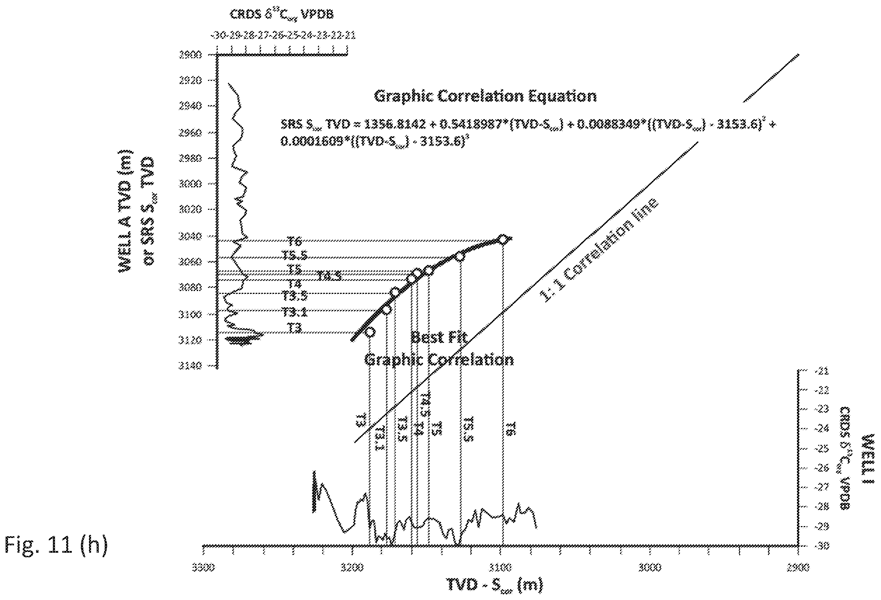

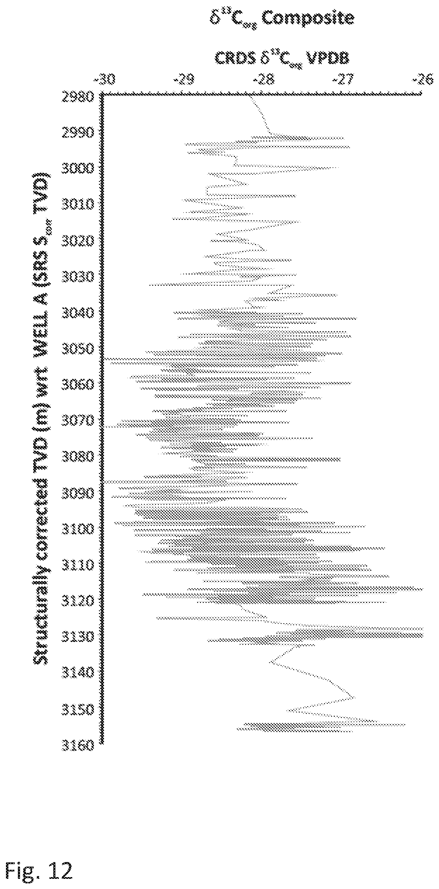

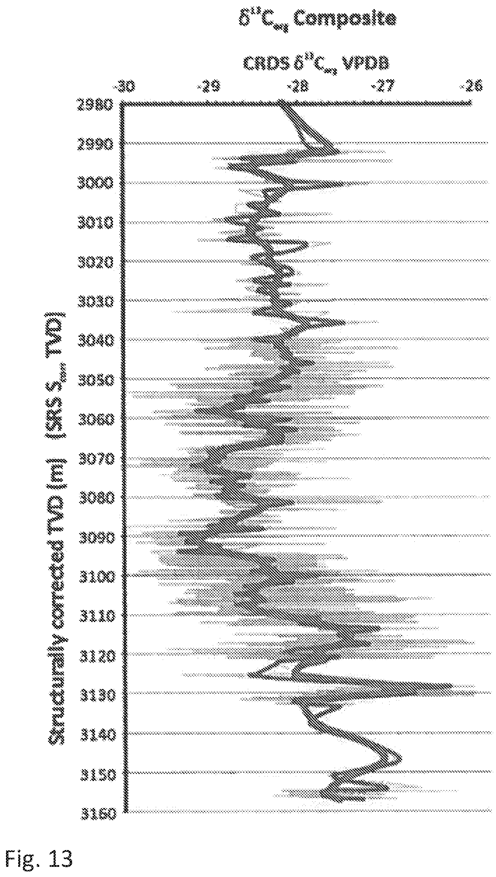

For example, Figures 9 (a) and (b) illustrate eight lateral well paths (as a function of TVD and MD) for eight wells in a petroliferous region. Figures 9 (a) and (b) also illustrate how the lateral well paths can be corrected to account for non-horizontality of the local geology. $\delta^{13}C_{org}$ values were obtained by CRDS of cuttings samples taken approximately every 30 feet along each well bore path. A ninth well was identified as the SRS, as shown in Figure 10. The correlation between each of the other wells and the SRS, as obtained using the graphic correlation method previously discussed, is shown in Figures 11 (a) to (h). A composite profile for the area, as shown in Figure 12, was obtained based on the correlations shown in Figures 11 (a) to (h). As shown in Figure 13, the composite profile data has been smoothed using a spline algorithm to create a simplified $\delta^{13}C_{org}$ composite profile, which enables chronostratigraphic trends across the region be identified more easily.

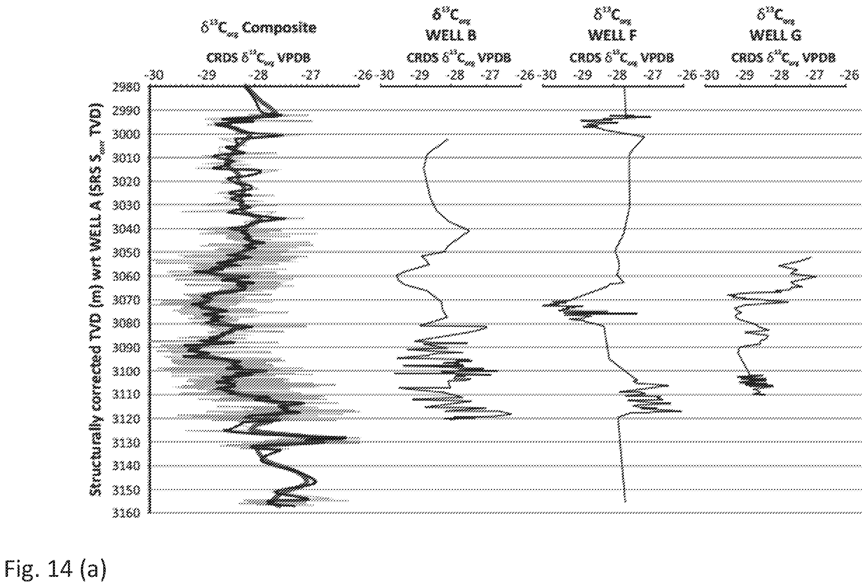

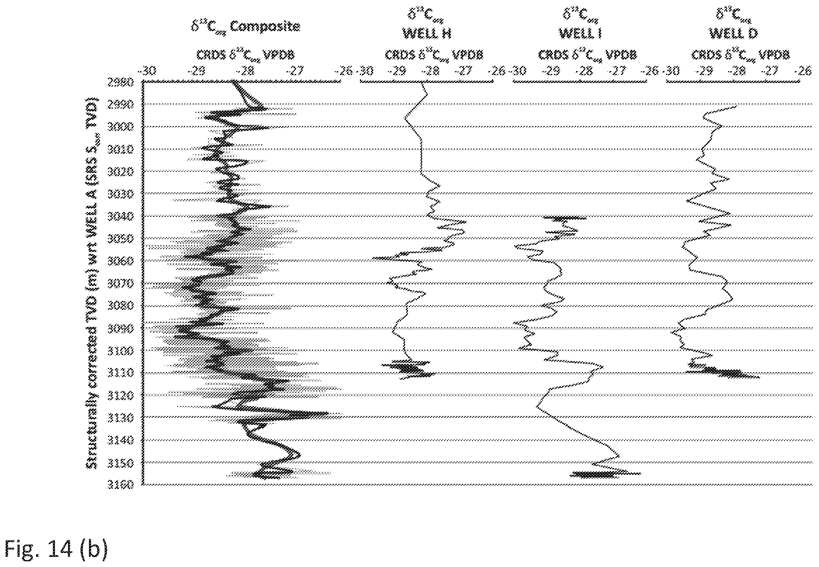

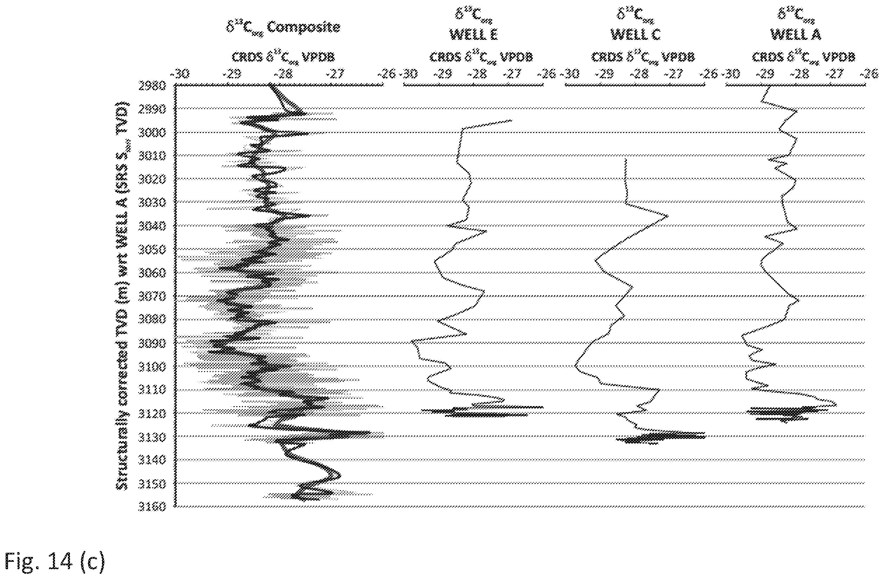

The $\delta^{13}C_{org}$ data for each well (relative to the corrected depth TVD - $S_{cor}$) is plotted side-by-side with the smoothed composite profile in Figures 14 (a) to (c). Since the depth values have been corrected to take into account lateral well bore slope, the corrected depth axis of Figures 14 (a) to (c) is equivalent to a time axis, with greater depths corresponding to older rock. The horizontal lines indicated in Figures 14 (a) to (c) are therefore isochronous surfaces which connect points in the various wells of the same age and bound packages of rock strata which are isochronous.

*Integration of geochemical data*

Geochemical data obtained from measurements made on samples (such as cuttings samples) taken from lateral wells can be integrated into the chronostratigraphic model developed by analysis of $\delta^{13}C_{org}$ data in order to construct chronostratigraphically constrained composite profiles and to generate high-resolution surface correlations along and between lateral wells.

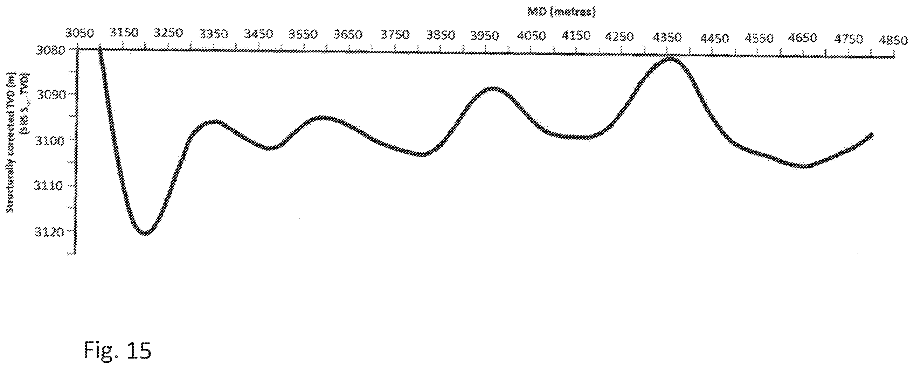

For example, Figure 15 illustrates an example lateral well path in terms of MD and the structurally corrected depth, TVD - $S_{cor}$, for the SRS. As the depth has been corrected for non-horizontality of the local geological structure and a chronostratigraphic correlation has been carried out for the lateral wells in the area, the TVD - $S_{cor}$ axis is effectively a time axis in chronostratigraphic units for the group of lateral wells. Accordingly, horizontal lines drawn in Figure 15 would be time lines connecting locations along the well bore of the same age.

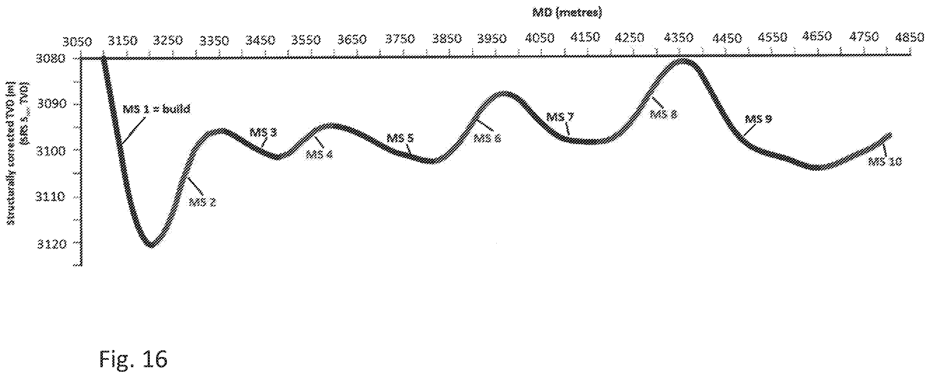

As shown in Figure 16, the well path is then divided into sections for carrying out a graphic correlation along the path itself, i.e. for identifying correlations between different sections of the well path. The skilled person will appreciate that the path may be divided up in different ways. For example, the sections may be defined by distance along the path, each section having a predetermined length (e.g., every 20 metres along the path). However, in the example shown in Figure 16, the path is divided into sections in which the sign of the gradient of the path is constant (i.e., sections which are always increasing or always decreasing in depth when travelling along the path from left to right). In Figure 16, these sections are labelled as MS 1, MS 2, MS 3, etc., where "MS" refers to "miniature vertical section". MS 1 corresponds to the build section of the lateral well which includes the vertical section of the path down to the deepest part of the well (i.e., the well heel). MS 2 extends from the well heel to the next inflection point along the lateral segment of the well, and so on.

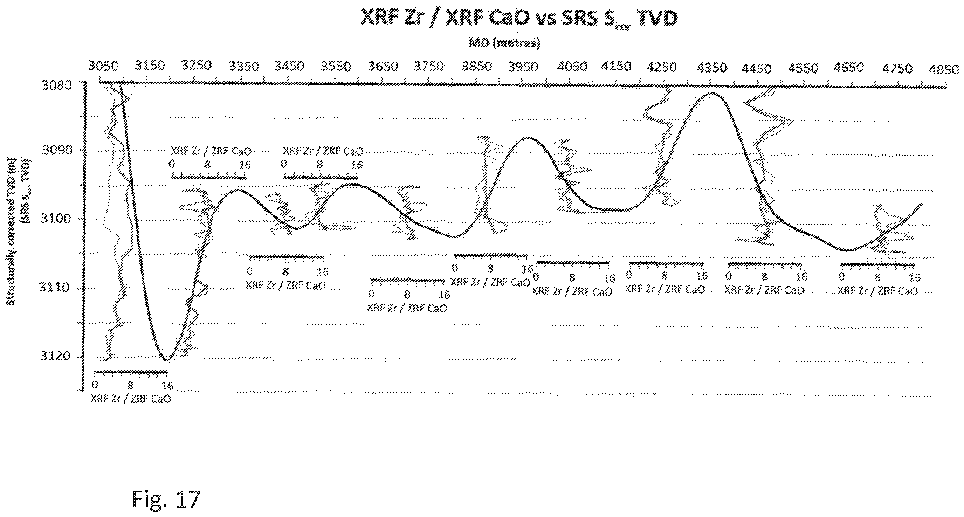

In order to identify correlations between each section, geochemical data is measured from cuttings samples obtained at multiple different locations along each section and this data is plotted against TVD - $S_{cor}$ for the SRS. For example, X-ray fluorescence (XRF) can be used to analyse the composition of cuttings samples. Certain components have previously been identified as sequence stratigraphic proxies for rock characteristics such as the presence of particular minerals or grain size. For example, grain size can be inferred from XRF-obtained Zr/CaO or Zr/Al ratios. Figure 17 illustrates XRF-data for Zr/CaO ratios measured from cuttings samples obtained across each section of the lateral segment of the well bore. The data for each section is plotted horizontally mid-way between the maximum and minimum TVD for the respective section. As can be seen in Figure 17, the data obtained for MS 1 (corresponding to the build section of the well bore) is relatively widely spaced (in terms of SRS TVD - $S_{cor}$) in comparison to the data obtained for the other sections. The skilled person will appreciate that the frequency of sample collection during drilling of the vertical build section of the well bore is typically lower than that in the lateral section of the well bore, as cuttings sample collection during drilling of the vertical build section will typically reduce the speed at which the landing zone depth can be reached safely.

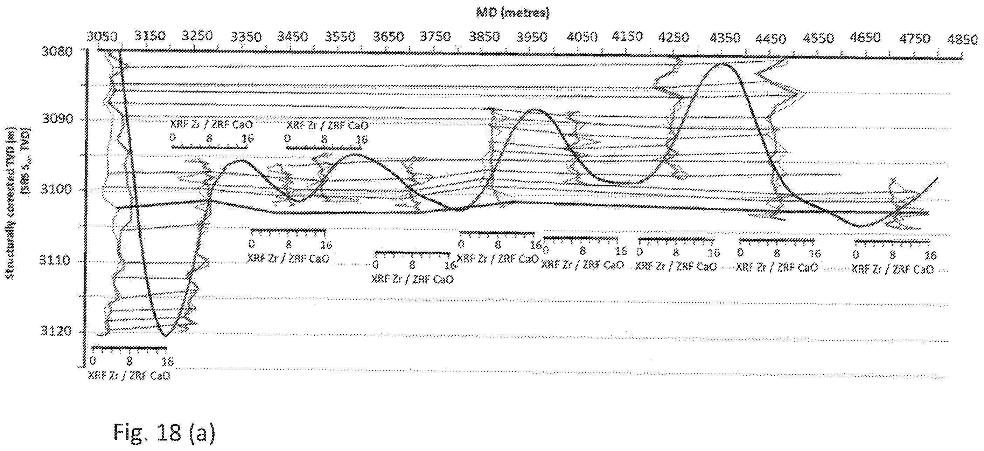

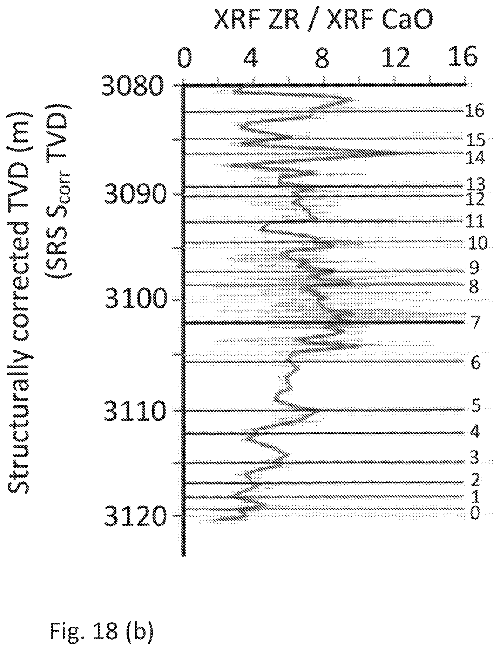

Correlations between the plots of the XRF data for the different sections can be determined from Figure 17, as illustrated in Figures 18 (a) and (b). However, as previously discussed, horizontal lines on Figures 17 and 18 are chronostratigraphic lines and, therefore, correlations between geochemical profiles for each section should not cross these lines. If significant crossing of lines is identified, this is an indication that the structural correction, $S_{cor}$, may need to be improved, for example using the average inclination of the new geochemical correlation lines. This can be achieved by: establishing a new correlation surface which extends along the length of the lateral section of the well; determining a new average inclination for the new correlation surface; and then repeating the structural correction calculation to determine a new $S_{cor}$, thereby moving the correlation line towards the horizontal. This process may be carried out iteratively, sequentially refining the graphical correlation in the process.

A composite profile for the lateral well may also be constructed, again as shown in Figure 18. Because the stratigraphic picks have been made along the lateral well on the basis of correlation lines between the geochemical sequence stratigraphic proxy profiles in MD, the stratigraphic picks can also be expressed in terms of the well's original TVD and MD.

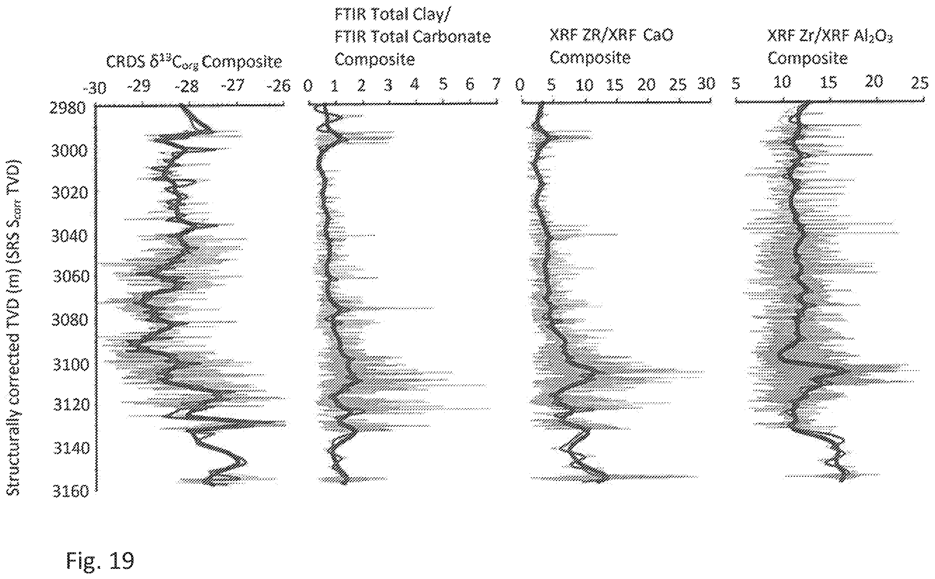

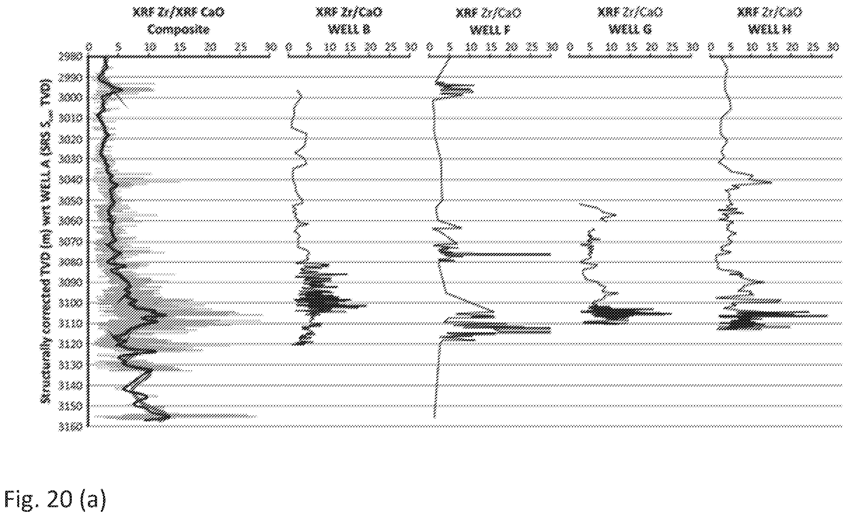

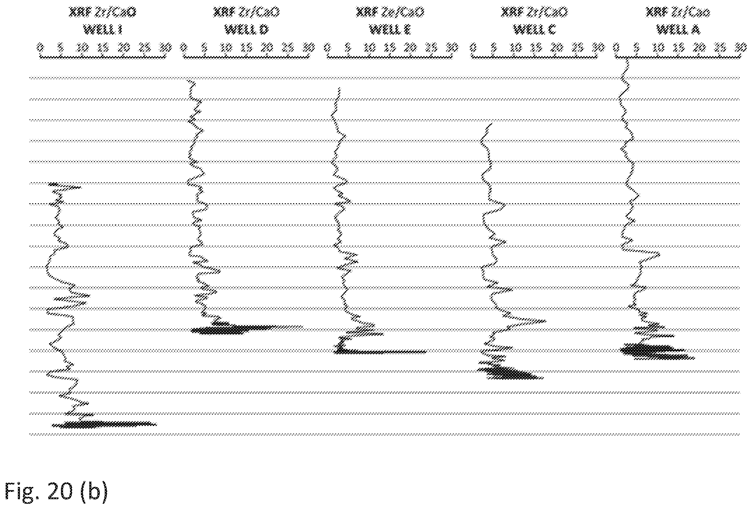

Composite profiles may be constructed for individual lateral wells and for a plurality of lateral wells in an area. Moreover, as illustrated in Figure 19, chronostratigraphically constrained composite profiles for various different geochemical parameters can be obtained. For example, Figure 19 illustrates spline-smoothed composite profiles for a plurality of lateral wells in a petroliferous region based on: $\delta^{13}C_{org}$; the ratio of total clay to total carbonate (obtained from Fourier-transform infrared spectroscopy (FTIR) measurements); the Zr/CaO ratio (obtained from XRF measurements); and the $Zr/Al_2O_3$ ratio (obtained from XRF measurements). Figures 20 (a) and (b) (Figure 20 (b) being a continuation of Figure 20 (a)) illustrate how Zr/CaO ratios for the various lateral wells can be correlated with the composite profile.

*Re-Os Dating*

The chronostratigraphic method described hereinabove enables *relative* dating of the rock strata. For example, although isotopic data such as $\delta^{18}C_{org}$ measurements relate to rock age, local changes in sedimentary accumulation rates throughout geologic time can stretch and/or squeeze the isotope profile, distorting the profile shape and reducing the ability to correlate reliably to calibrated isotope profiles However, a sequence stratigraphic model which identifies the *absolute* ages of the rock strata is particularly useful because it enables sediment accumulation rates to be calculated and studied across a basin. This is helpful in hydrocarbon exploration because, for example, it is known that mudrocks having high total organic carbon levels typically form under conditions of slow accumulation rates. The mechanical properties of a rock which, for example, determine whether the rock is suitable for hydraulic fracturing, may also depend on sedimentation rates. The accumulation rate distortion of a profile can be removed by use of, for example, unique biostratigraphy or other absolute age measurements to constrain the profile and enable accurate plotting of the profile relative to absolute time.

One method of determining the absolute age of a rock sample is rhenium-osmium (Re-Os) dating. Re-Os dating is a form of radiometric dating based on the beta decay of the isotope $^{187}$Re to [187]Os. Re and Os are both siderophilic (i.e., iron-loving) elements. Re is also a chalcophilic (i.e., sulfur-loving) element. Re-Os dating is particularly useful for dating sediments deposited under suboxic, anoxic or euxinic (i.e. anoxic and sulfidic) conditions, of which oil shales are one example. This is because, under oxic conditions, both Re and Os are soluble in sea water, while under anoxic conditions, Re and Os become insoluble and, therefore, sediment at the seafloor/seawater interface becomes enriched in hydrogeneous Re and Os. Accordingly, the ratios of Re and Os isotopes in sedimentary rock reflect the seawater conditions at the time of deposition. When the sediment is cut off from interacting with anoxic seawater, the Re-Os system in the rock becomes closed and the isotopic clock commences recording the age of deposition.

Re-Os dating is conventionally carried out on the basis of a whole rock isochron dating method, meaning that no assumptions about the initial amount of the daughter $^{187}$Os nuclide in a sample are required. It is instead assumed that, at the time of its formation, the rock contained unknown amounts of both radiogenic Os ($^{187}$Os) and non-radiogenic Os ($^{188}$Os), along with some amount of the parent $^{187}$Re. Accordingly, at the time of rock formation, the ratio of the concentration of $^{187}$Os to the concentration of $^{188}$Os was some value independent of the concentration of $^{187}$Re. It is assumed that, as time passed, some amount of $^{187}$Re decayed into $^{187}$Os, increasing the $^{187}$Os/$^{188}$Os ratio. The greater the initial concentration of $^{187}$Re, the greater the concentration of $^{187}$Os at some particular future time. Therefore, the $^{187}$Os/$^{188}$Os ratio becomes larger with time, while the $^{187}$Re/$^{187}$Os ratio becomes smaller with time. The $^{187}$Os/$^{188}$Os ratio will change more quickly with time for rocks which started out with larger concentrations of $^{187}$Re than rocks which started out with smaller concentrations of $^{187}$Re.

Accordingly, for a given sample, the number of atoms (or concentration) of the radiogenic daughter isotope, $D^*$, at a time, $t$, is given by the equation:

$$D^* = D_0 + n\left(e^{\lambda t} - 1\right) \tag{8}$$

where $D_0$ is the number of atoms (or concentration) of the radiogenic daughter isotope in the initial composition of the sample, n is the number of atoms (or concentration) of the parent isotope in the sample at time $t$, and $\lambda$ is the decay constant of the parent isotope. This equation may also be expressed as:

$$\left(\frac{D^*}{D_{ref}}\right) = \left(\frac{D_0}{D_{ref}}\right) + \left(\frac{P_t}{D_{ref}}\right)\left(e^{\lambda t} - 1\right) \tag{9}$$

Where $D_{ref}$ is the number of atoms (or concentration) of the non-radiogenic isotope of the daughter element in the sample (assumed to be constant) and $P_t$ is the number of atoms (or concentration) of the parent isotope which has decayed over time $t$.

In whole rock isochron dating, a selection of different sample rocks are taken from a common reservoir. The ratios ($\frac{D^*}{D_{ref}}$) and ($\frac{P_t}{D_{ref}}$) can be determined for each rock, for example by mass spectrometry, and are then plotted against one another in what is called an isochron plot. A best-fit line (called an isochron) is drawn through the data. The slope of the line, ($e^{\lambda t}$ - represents the ratio of daughter isotope to parent isotope in standard radiometric dating and can therefore be used to calculate the age of the reservoir at time $t$. The intercept with the ($\frac{D^*}{D_{ref}}$) axis yields the initial radiogenic daughter ratio, ($\frac{D_0}{D_{ref}}$).

Re-Os dating is typically carried out on core samples which, as discussed hereinabove, are time-consuming and expensive to obtain. The present inventors, however, have developed the following methodology which enables Re-Os dating of cuttings samples taken from wells.

Re-Os isotopic measurements are made on spot cuttings taken from a well, the spot cuttings being collected over, for example, about 6 metres of the well path at a sampling spacing of about 1 metre. Below a spacing of about 1 metre, mixing of the spot cuttings tends to take place as the drilling mud is circulated up out of the well, leading to overlap between results for adjacent sampling locations. Re and Os isotopic ratios are determined using negative thermal ionization mass spectrometry of samples following Carius tube dissolution, solvent extraction and ion exchange. Each spot cuttings sample is analysed twice. The $^{187}$Os/$^{188}$Os and $^{187}$Re/$^{188}$Os ratios are calculated for each sample and plotted together to enable an isochron to be determined.

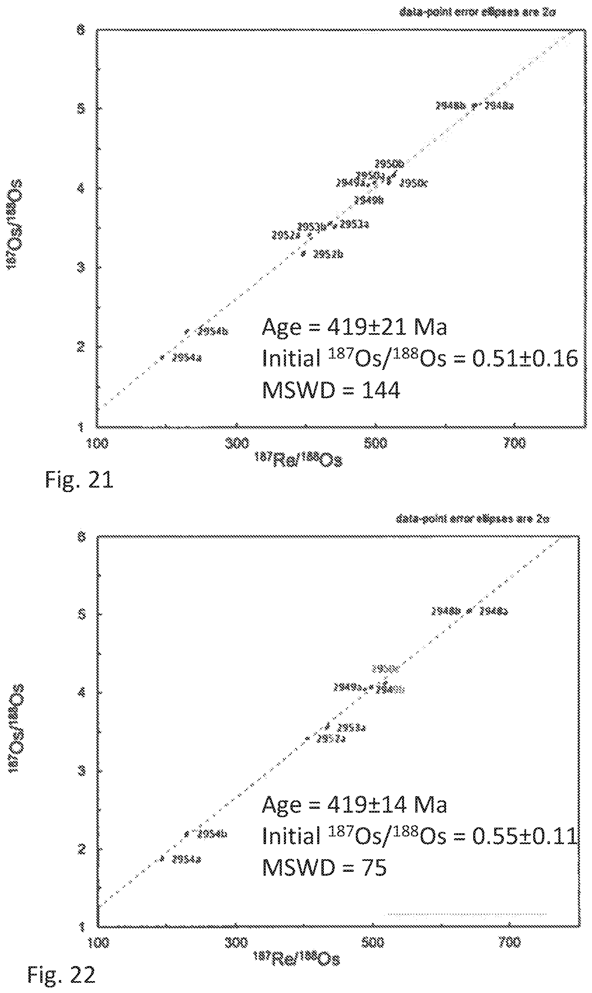

For example, Figure 21 illustrates the isochron obtained from measurements made on thirteen cuttings samples. This isochron yields an age of 419 ± 21 Ma (i.e., about 5 % uncertainty), assuming a $^{187}$Re decay constant of $1.666e^{-11}a^{-1}$, with the uncertainty quoted at the $2\sigma$ level. As can be seen in Figure 21, the mean square weighted deviation (MSWD) in the fit, equivalent to a reduced chi-squared statistic, is 144. An MSWD significantly greater than 1 indicates that the isochron fit has not fully captured the experimental data. Refinement of the model is therefore necessary.

The present inventors have identified that the large MSWD seen in such calculations is the result of different samples having different initial $^{187}$Os/$^{188}$Os ratios, whereas uniform initial $^{187}$Os/$^{188}$Os ratios is a fundamental assumption of Re-Os dating. In order to correct for this, the samples can be re-grouped systematically according to the initial $^{187}$Os/$^{188}$Os isotope compositions. The initial $^{187}$Os/$^{188}$Os isotope composition for a given sample can be calculated by rearranging Equation (9) and assuming a particular value for the age of the rock unit (for example, the age determined from the initial isochron including all samples or an age determined using other methods, such as uranium-lead (U-Pb) dating). The samples can be arranged in stratigraphic order and then a group of samples, which are stratigraphically close to one another and similar in terms of initial $^{187}$Os/$^{188}$Os isotope composition, is selected to plot a new isochron. For example, Figure 22 shows the isochron produced from those cuttings samples of Figure 21 having an initial $^{187}$Os/$^{188}$Os ratio of 0.55 $\pm$ 0.11, which provides an age of 419 $\pm$ 14 Ma (i.e., about 3 % uncertainty) and an MSWD of 75. Accordingly, by restricting the isochron analysis to those samples having similar initial $^{187}$Os/$^{188}$Os ratio, the accuracy of the predicted age is improved.

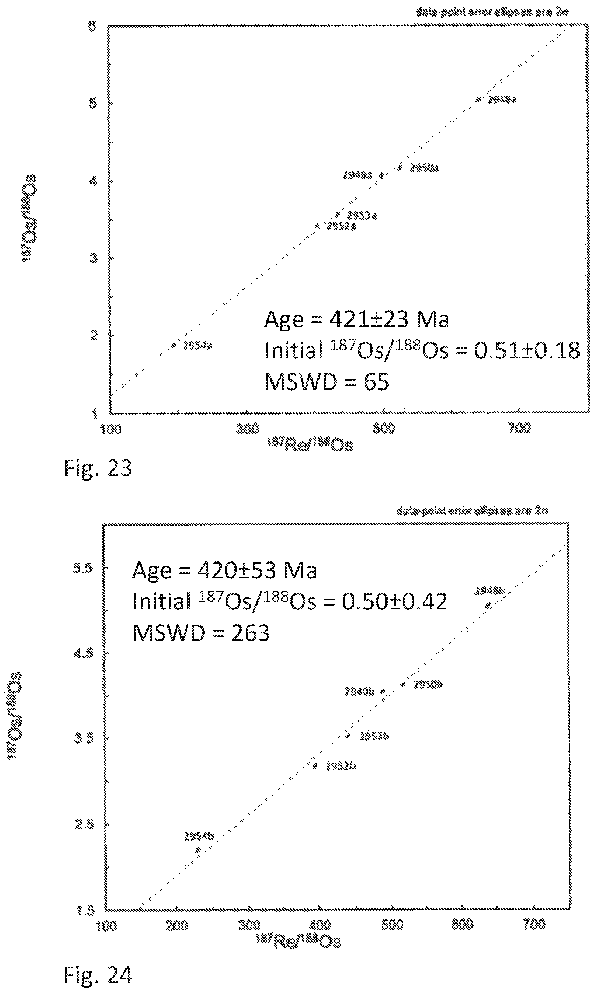

Moreover, Figures 23 and 24 illustrate the isochrons produced if measurements are not replicated for each cuttings sample. In particular, the samples labelled "a" and "b" in Figures 21 and 22 relate to measurements made on "a" and "b" picks from the same volume of cuttings. The isochron in Figure 23 is based only on the "a" replicas and the isochron in Figure 24 is based only on the "b" replicas. Figure 23 yields an age of 421 $\pm$ 23 Ma (i.e., about 5 % uncertainty) and an MSWD of 65. However, Figure 24 yields an age of 420 $\pm$ 53 Ma (i.e., about 13 % uncertainty) and an MSWD of 263. This indicates that replicate analyses of samples at each cuttings interval are important in order to achieve accurate and consistent Re-Os dating results. This is necessary because each "pick" from a volume of cuttings is likely to have a slightly different composition, given that cuttings are sampled across a particular depth interval and not at one specific depth. The inventors have found that the variation in Re and Os isotope concentrations across a single cuttings sampling depth interval is significantly greater than that typically found in whole element chemostratigraphy.

Accordingly, accurate rock ages can be determined based on Re-Os dating of cuttings samples when replicate analyses are carried out and samples are systematically grouped by initial $^{187}$Os/$^{188}$Os ratios.

It will be understood that the invention is not limited to the embodiments described above and various modifications and improvements can be made without departing from the concepts described herein.

## Claims

1. A method comprising determining stratigraphic correlations between two or more sets of stratigraphic measurements, each set of stratigraphic measurements being obtained from a different lateral well in a region, wherein:

   (i) each set of stratigraphic measurements comprises:

      (a) measurements of a stratigraphic parameter obtained from a plurality of rock samples from the respective lateral well, each rock sample of the respective plurality of rock samples being associated with a different measurement location within the respective lateral well; and
      (b) corresponding measurements of a depth parameter for each measurement location in the respective lateral well,

   wherein the stratigraphic parameter is a parameter indicative of a rock characteristic and the depth parameter is a parameter indicative of a subterranean depth of a measurement location; and
   (ii) the method further comprises, prior to determining stratigraphic correlations between the two or more sets of stratigraphic measurements, correcting one or more of the sets of stratigraphic measurements to take into account a non-zero inclination of the respective lateral well relative to the local ground surface, wherein the measured depth parameter for each measurement location is indicative of a subterranean depth of the said measurement location relative to the local ground surface, and wherein correcting a set of stratigraphic measurements to take into account a non-zero inclination of the respective lateral well relative to the local ground surface comprises:

      determining the inclination of the lateral well relative to the local ground surface; and
      for each measurement location in the lateral well, calculating a corrected depth parameter, indicative of a depth of the measurement location relative to a local geological horizon, taking into account the determined inclination.

2. The method according to claim 1, wherein:

(i) the rock samples are cuttings samples; and/or

(ii) the method further comprises, prior to determining stratigraphic correlations between the two or more sets of stratigraphic measurements, measuring values of the stratigraphic parameter, and corresponding values of the depth parameter, for each of the plurality of rock samples.

3. The method according to claim 1 or claim 2, wherein the inclination of the lateral well is the average inclination of a lateral segment of the lateral well.

4. The method according to claim 3, wherein the average inclination of a lateral segment of the lateral well is the inclination of an average straight line path for the lateral segment of the lateral well, optionally wherein determining the average inclination of the lateral segment of the lateral well comprises:

fitting a straight line to the path followed by the lateral segment of the lateral well; and

determining the inclination of the straight line relative to the local ground surface;

further optionally wherein fitting the straight line and determining the inclination is achieved by plotting the lateral segment of the lateral well as a function of (a) vertical depth below the local ground level and (b) lateral distance travelled along the well bore and fitting a straight line to the plotted lateral segment.

5. The method according to any preceding claim, wherein:

(a) the method comprises determining stratigraphic correlations between the two or more sets of stratigraphic measurements by a graphic correlation method;

(b) the two or more sets of stratigraphic measurements are two or more first sets of stratigraphic measurements each comprising measurements of a first stratigraphic parameter and the method further comprises determining stratigraphic correlations between the two or more first sets of stratigraphic measurements and two or more second sets of stratigraphic measurements, the two or more second sets of stratigraphic measurements each comprising measurements of a second stratigraphic parameter different from the first stratigraphic parameter, optionally wherein the two or more second sets of stratigraphic measurements are obtained from the same lateral wells in the region as the two or more first sets of stratigraphic measurements;

(c) the or each stratigraphic parameter is:

a chronostratigraphic parameter indicative of relative rock age or absolute rock age, for example a radiometric dating parameter or a stable isotope dating parameter;

a chemostratigraphic parameter indicative of rock composition; and/or

a lithostratigraphic parameter indicative of rock type;

(d) the method further comprises compiling a stratigraphic composite profile for the region based on the identified correlations;

(e) the method further comprises constructing a sedimentary depositional model of the region based on the identified correlations; and/or

(f) the method further comprises targeting a sub-region of the region for hydrocarbon exploration based on the identified correlations, optionally wherein the method comprises drilling a lateral well for hydrocarbon extraction in the targeted sub-region.

6. The method according to any preceding claim, wherein the steps of claims 1, 5 (a), 5 (b), 5 (d), 5 (e) or 5 (f) are carried out by a computer.

7. A method comprising determining stratigraphic correlations between two or more sets of stratigraphic measurements, each set of stratigraphic measurements being obtained from different sections of the same lateral well in a region, wherein:

(i) each set of stratigraphic measurements comprises:

(a) measurements of a stratigraphic parameter obtained from a plurality of rock samples from the respective section of the lateral well, each rock sample of the respective plurality of rock samples being associated with a different measurement location within the respective section of the lateral well; and

(b) corresponding measurements of a depth parameter for each measurement location in the respective section of the lateral well,

wherein the stratigraphic parameter is a parameter indicative of a rock characteristic and the depth parameter is a parameter indicative of a subterranean depth of a measurement location; and

(ii) the method further comprises, prior to determining stratigraphic correlations between the two or more sets of stratigraphic measurements, correcting the two or more sets of stratigraphic measurements to take into account a non-zero inclination of the lateral well relative to the local ground surface,

wherein the measured depth parameter for each measurement location is indicative of a subterranean depth of the said measurement location relative to the local ground surface, and wherein correcting a set of stratigraphic measurements to take into account a non-zero inclination of the lateral well relative to the local ground surface comprises:

> determining the inclination of the lateral well relative to the local ground surface; and
> for each measurement location in the lateral well, calculating a corrected depth parameter, indicative of a depth of the measurement location relative to a local geological horizon, taking into account the determined inclination.

8. The method according to claim 7, wherein:

> (i) the different sections of the same lateral well are different sections of a lateral segment of the same lateral well;
> (ii) the rock samples are cuttings samples; and/or
> (iii) the method further comprises, prior to determining stratigraphic correlations between the two or more sets of stratigraphic measurements, measuring values of the stratigraphic parameter, and corresponding values of the depth parameter, for each of the plurality of rock samples.

9. The method according to claim 7 or claim 8, wherein the inclination of the lateral well is the average inclination of a lateral segment of the lateral well.

10. The method according to claim 9, wherein the average inclination of a lateral segment of the lateral well is the inclination of an average straight line path for the lateral segment of the lateral well, optionally wherein determining the average inclination of the lateral segment of the lateral well comprises:

> fitting a straight line to the path followed by the lateral segment of the lateral well; and
> determining the inclination of the straight line relative to the local ground surface;
> further optionally wherein fitting the straight line and determining the inclination is achieved by plotting the lateral segment of the lateral well as a function of (a) vertical depth below the local ground level and (b) lateral distance travelled along the well bore and fitting a straight line to the plotted lateral segment.

11. The method according to any of claims 7 to 10, wherein the steps of claim 7 are carried out by a computer.

12. A computer program comprising instructions which, when the program is executed by a computer, cause the computer to carry out the steps of the method of: claims 1, 5 (a), 5 (b), 5 (d), 5 (e) or 5 (f); or claim 7.

13. A computer-readable medium storing the computer program according to claim 12.

**Patentansprüche**

1. Verfahren, das das Bestimmen stratigraphischer Korrelationen zwischen zwei oder mehr Sätzen stratigraphischer Messungen umfasst, wobei jeder Satz stratigraphischer Messungen von einem anderen lateralen Bohrloch in einer Region erhalten wird, wobei:

> (i) jeder Satz stratigraphischer Messungen Folgendes umfasst:

>> (a) Messungen eines stratigraphischen Parameters, der aus mehreren Gesteinsproben aus dem jeweiligen lateralen Bohrloch erhalten wird, wobei jede Gesteinsprobe der jeweiligen Vielzahl von Gesteinsproben mit einem anderen Messort innerhalb des jeweiligen lateralen Bohrlochs assoziiert ist; und
>> (b) entsprechende Messungen eines Tiefenparameters für jeden Messort in dem jeweiligen lateralen Bohrloch,

wobei der stratigraphische Parameter ein Parameter ist, der eine Gesteinscharakteristik angibt, und der Tiefenparameter ein Parameter ist, der eine unterirdische Tiefe eines Messorts angibt; und
(ii) das Verfahren umfasst ferner, vor dem Bestimmen stratigraphischer Korrelationen zwischen den zwei oder mehr Sätzen stratigraphischer Messungen, Korrigieren eines oder mehrerer der Sätze stratigraphischer Messungen, um eine Neigung des jeweiligen lateralen Bohrlochs relativ zu der lokalen Bodenoberfläche von ungleich Null zu berücksichtigen,

wobei der gemessene Tiefenparameter für jeden Messort eine unterirdische Tiefe des Messorts relativ zu der lokalen Bodenoberfläche angibt, und wobei das Korrigieren eines Satzes von stratigraphischen Messungen, um eine Neigung des jeweiligen lateralen Bohrlochs relativ zu der lokalen Bodenoberfläche von ungleich Null zu berücksichtigen, Folgendes umfasst:

Bestimmen der Neigung des lateralen Bohrlochs relativ zu der lokalen Bodenfläche; und
Berechnen eines korrigierten Tiefenparameters für jeden Messort in dem lateralen Bohrloch, der eine Tiefe des Messorts relativ zu einem lokalen geologischen Horizont angibt, unter Berücksichtigung der bestimmten Neigung.

2. Verfahren nach Anspruch 1, wobei:

(i) die Gesteinsproben Schnittproben sind; und/oder
(ii) das Verfahren ferner vor dem Bestimmen stratigraphischer Korrelationen zwischen den zwei oder mehr Sätzen stratigraphischer Messungen Messwerte des stratigraphischen Parameters und entsprechende Werte des Tiefenparameters für jede der mehreren Gesteinsproben umfasst.

3. Verfahren nach Anspruch 1 oder Anspruch 2, wobei die Neigung des lateralen Bohrlochs die durchschnittliche Neigung eines lateralen Segments des lateralen Bohrlochs ist.

4. Verfahren nach Anspruch 3, wobei die durchschnittliche Neigung eines lateralen Segments des lateralen Bohrlochs die Neigung eines durchschnittlichen geradlinigen Pfads für das laterale Segment des lateralen Bohrlochs ist, wobei optional das Bestimmen der durchschnittlichen Neigung des lateralen Segments des lateralen Bohrlochs Folgendes umfasst:

Anbringen einer geraden Linie an dem Pfad, gefolgt von dem lateralen Segment des lateralen Bohrlochs; und
Bestimmen der Neigung der Geraden relativ zu der lokalen Bodenfläche;
ferner optional, wobei das Anpassen der Geraden und das Bestimmen der Neigung durch Auftragen des lateralen Segments des lateralen Bohrlochs als eine Funktion von (a) vertikaler Tiefe unterhalb des lokalen Bodenniveaus und (b) lateraler Entfernung, die entlang des Bohrlochs zurückgelegt wird, und Anpassen einer Geraden an das aufgezeichnete laterale Segment erreicht wird.

5. Verfahren nach einem der vorhergehenden Ansprüche, wobei:

(a) das Verfahren Bestimmen stratigraphischer Korrelationen zwischen den zwei oder mehr Sätzen stratigraphischer Messungen durch ein graphisches Korrelationsverfahren umfasst;
(b) die zwei oder mehr Sätze von stratigraphischen Messungen zwei oder mehr erste Sätze von stratigraphischen Messungen sind, die jeweils Messungen eines ersten stratigraphischen Parameters umfassen, und das Verfahren ferner die Bestimmung von stratigraphischen Korrelationen zwischen den zwei oder mehr ersten Sätzen von stratigraphischen Messungen und zwei oder mehr zweiten Sätzen von stratigraphischen Messungen umfasst, wobei die zwei oder mehr zweiten Sätze von stratigraphischen Messungen jeweils Messungen eines zweiten stratigraphischen Parameters umfassen, der sich von dem ersten stratigraphischen Parameter unterscheidet, wobei die zwei oder mehr zweiten Sätze von stratigraphischen Messungen optional aus denselben lateralen Bohrlöchern in der Region wie die zwei oder mehr ersten Sätze von stratigraphischen Messungen erhalten werden;
(c) der oder jeder stratigraphische Parameter Folgendes ist:

ein chronostratigraphischer Parameter, der ein relatives Gesteinsalter oder ein absolutes Gesteinsalter angibt, zum Beispiel einen radiometrischen Datierungsparameter oder einen stabilen Isotopendatingparameter;
ein chemostratigraphischer Parameter, der eine Gesteinszusammensetzung anzeigt; und/oder ein lithostratigraphischer Parameter, der den Gesteinstyp angibt;

(d) das Verfahren ferner Kompilieren eines stratigraphischen Verbundprofils für die Region basierend auf den identifizierten Korrelationen umfasst;

(e) das Verfahren ferner das Konstruieren eines sedimentären Ablagerungsmodells der Region basierend auf den identifizierten Korrelationen umfasst; und/oder

(f) das Verfahren ferner das Targeting einer Teilregion der Region zur Kohlenwasserstoffexploration basierend auf den identifizierten Korrelationen umfasst, wobei das Verfahren optional das Bohren eines lateralen Bohrlochs zur Kohlenwasserstoffextraktion in der Zielteilregion umfasst.

6. Verfahren nach einem der vorhergehenden Ansprüche, wobei die Schritte der Ansprüche 1, 5 (a), 5 (b), 5 (d), 5 (e) oder 5 (f) durch einen Computer ausgeführt werden.

7. Verfahren, das das Bestimmen stratigraphischer Korrelationen zwischen zwei oder mehr Sätzen stratigraphischer Messungen umfasst, wobei jeder Satz stratigraphischer Messungen von verschiedenen Abschnitten desselben lateralen Bohrlochs in einer Region erhalten wird, wobei:

(i) jeder Satz stratigraphischer Messungen Folgendes umfasst:

(a) Messungen eines stratigraphischen Parameters, der aus einer Vielzahl von Gesteinsproben aus dem jeweiligen Abschnitt des lateralen Bohrlochs erhalten wird, wobei jede Gesteinsprobe der jeweiligen Vielzahl von Gesteinsproben mit einem anderen Messort innerhalb des jeweiligen Abschnitts des lateralen Bohrlochs assoziiert ist; und

(b) entsprechende Messungen eines Tiefenparameters für jeden Messort in dem jeweiligen Abschnitt des lateralen Bohrlochs,

wobei der stratigraphische Parameter ein Parameter ist, der eine Gesteinscharakteristik angibt, und der Tiefenparameter ein Parameter ist, der eine unterirdische Tiefe eines Messorts angibt; und

(ii) das Verfahren ferner vor dem Bestimmen stratigraphischer Korrelationen zwischen den zwei oder mehr Sätzen stratigraphischer Messungen, Korrigieren der zwei oder mehr Sätze stratigraphischer Messungen umfasst, um eine Neigung des lateralen Bohrlochs relativ zu der lokalen Bodenoberfläche von ungleich Null zu berücksichtigen,

wobei der gemessene Tiefenparameter für jeden Messort eine unterirdische Tiefe des Messorts relativ zu der lokalen Bodenoberfläche angibt, und wobei das Korrigieren eines Satzes von stratigraphischen Messungen, um eine Neigung des lateralen Bohrlochs relativ zu der lokalen Bodenoberfläche von ungleich Null zu berücksichtigen, Folgendes umfasst:

Bestimmen der Neigung des lateralen Bohrlochs relativ zu der lokalen Bodenfläche; und
Berechnen eines korrigierten Tiefenparameters für jeden Messort in dem lateralen Bohrloch, der eine Tiefe des Messorts relativ zu einem lokalen geologischen Horizont angibt, unter Berücksichtigung der bestimmten Neigung.

8. Verfahren nach Anspruch 7, wobei:

(i) die verschiedenen Abschnitte desselben lateralen Bohrlochs unterschiedliche Abschnitte eines seitlichen Segments desselben lateralen Bohrlochs sind;
(ii) die Gesteinsproben Schnittproben sind; und/oder
(iii) das Verfahren ferner vor dem Bestimmen stratigraphischer Korrelationen zwischen den zwei oder mehr Sätzen stratigraphischer Messungen Messwerte des stratigraphischen Parameters und entsprechende Werte des Tiefenparameters für jede der mehreren Gesteinsproben umfasst.

9. Verfahren nach Anspruch 7 oder Anspruch 8, wobei die Neigung des lateralen Bohrlochs die durchschnittliche Neigung eines lateralen Segments des lateralen Bohrlochs ist.

10. Verfahren nach Anspruch 9, wobei die durchschnittliche Neigung eines lateralen Segments des lateralen Bohrlochs die Neigung eines durchschnittlichen geradlinigen Pfads für das laterale Segment des lateralen Bohrlochs ist, wobei optional das Bestimmen der durchschnittlichen Neigung des lateralen Segments des lateralen Bohrlochs Folgendes umfasst:

Anbringen einer geraden Linie an dem Pfad, gefolgt von dem lateralen Segment des lateralen Bohrlochs; und
Bestimmen der Neigung der Geraden relativ zu der lokalen Bodenfläche;
ferner optional, wobei das Anpassen der Geraden und das Bestimmen der Neigung durch Auftragen des lateralen Segments des lateralen Bohrlochs als eine Funktion von (a) vertikaler Tiefe unterhalb des lokalen Bodenniveaus und (b) lateraler Entfernung, die entlang des Bohrlochs zurückgelegt wird, und Anpassen einer Geraden an das aufgezeichnete laterale Segment erreicht wird.

11. Verfahren nach einem der Ansprüche 7 bis 10, wobei die Schritte nach Anspruch 7 durch einen Computer ausgeführt werden.

12. Computerprogramm, das Anweisungen umfasst, die, wenn das Programm durch einen Computer ausgeführt wird, den Computer veranlassen, die Schritte des Verfahrens nach den Ansprüchen 1, 5 (a), 5 (b), 5 (d), 5 (e) oder 5 (f) auszuführen; oder Anspruch 7.

13. Computerlesbares Medium, das das Computerprogramm nach Anspruch 12 speichert.


**Revendications**

1. Procédé comprenant la détermination de corrélations stratigraphiques entre deux ensembles ou plus de mesures stratigraphiques, chaque ensemble de mesures stratigraphiques étant obtenu à partir d'un puits latéral différent dans une région :

   (i) chaque ensemble de mesures stratigraphiques comprenant :

   (a) des mesures d'un paramètre stratigraphique obtenu à partir d'une pluralité d'échantillons de roche provenant du puits latéral respectif, chaque échantillon de roche de la pluralité respective d'échantillons de roche étant associé à un emplacement de mesure différent à l'intérieur du puits latéral respectif ; et
   (b) des mesures correspondantes d'un paramètre de profondeur pour chaque emplacement de mesure dans le puits latéral respectif,

   le paramètre stratigraphique étant un paramètre indicatif d'une caractéristique de la roche et le paramètre de profondeur étant un paramètre indicatif d'une profondeur souterraine d'un emplacement de mesure ; et
   (ii) le procédé comprenant en outre, avant la détermination des corrélations stratigraphiques entre les deux ensembles ou plus de mesures stratigraphiques, la correction d'un ou plusieurs des ensembles de mesures stratigraphiques pour prendre en compte une inclinaison non nulle du puits latéral respectif par rapport à la surface locale du sol,

   le paramètre de profondeur mesurée pour chaque emplacement de mesure étant indicatif d'une profondeur souterraine dudit emplacement de mesure par rapport à la surface locale du sol, et la correction d'un ensemble de mesures stratigraphiques pour prendre en compte une inclinaison non nulle du puits latéral respectif par rapport à la surface locale du sol comprenant :

   la détermination de l'inclinaison du puits latéral par rapport à la surface locale du sol ; et
   pour chaque emplacement de mesure dans le puits latéral, le calcul d'un paramètre de profondeur corrigé, indicatif d'une profondeur de l'emplacement de mesure par rapport à un horizon géologique local, compte tenu de l'inclinaison déterminée.

2. Procédé selon la revendication 1 :

   (i) les échantillons de roche étant des échantillons de déblais ; et/ou
   (ii) le procédé comprenant en outre, avant la détermination de corrélations stratigraphiques entre les deux ensembles ou plus de mesures stratigraphiques, la mesure de valeurs du paramètre stratigraphique et de valeurs correspondantes du paramètre de profondeur, pour chacun de la pluralité d'échantillons de roche.

3. Procédé selon la revendication 1 ou la revendication 2, l'inclinaison du puits latéral étant l'inclinaison moyenne d'un segment latéral du puits latéral.

4. Procédé selon la revendication 3, l'inclinaison moyenne d'un segment latéral du puits latéral étant l'inclinaison d'un trajet en ligne droite moyen pour le segment latéral du puits latéral, la détermination de l'inclinaison moyenne du segment latéral du puits latéral comprenant éventuellement :

l'ajustement d'une droite à la trajectoire suivie par le segment latéral du puits latéral ; et
la détermination de l'inclinaison de la droite par rapport à la surface locale du sol ;
en outre, l'ajustement de la ligne droite et la détermination de l'inclinaison étant éventuellement réalisés en traçant le segment latéral du puits latéral en fonction (a) de la profondeur verticale au-dessous du niveau local du sol et (b) de la distance latérale parcourue le long du puits de forage et en ajustant une droite au segment latéral tracé.

5. Procédé selon l'une quelconque des revendications précédentes :

(a) le procédé comprenant la détermination de corrélations stratigraphiques entre les deux ensembles ou plus de mesures stratigraphiques par une méthode de corrélation graphique ;
(b) les deux ensembles ou plus de mesures stratigraphiques étant deux premiers ensembles ou plus de mesures stratigraphiques comprenant chacun des mesures d'un premier paramètre stratigraphique et le procédé comprenant en outre la détermination de corrélations stratigraphiques entre les deux premiers ensembles ou plus de mesures stratigraphiques et deux seconds ensembles ou plus de mesures stratigraphiques, les deux seconds ensembles ou plus de mesures stratigraphiques comprenant chacun des mesures d'un second paramètre stratigraphique différent du premier paramètre stratigraphique, les deux ou plusieurs seconds ensembles de mesures stratigraphiques étant éventuellement obtenus à partir des mêmes puits latéraux dans la région que les deux premiers ensembles ou plus de mesures stratigraphiques ;
(c) le ou chaque paramètre stratigraphique étant :

un paramètre chronostratigraphique indicatif de l'âge relatif de la roche ou de l'âge absolu de la roche, par exemple un paramètre de datation radiométrique ou un paramètre de datation par isotope stable ;
un paramètre chimio-stratigraphique indiquant la composition de la roche ; et/ou
un paramètre lithostratigraphique indicatif du type de roche ;

(d) le procédé comprenant en outre la compilation d'un profil composite stratigraphique pour la région sur la base des corrélations identifiées ;
(e) le procédé comprenant en outre la construction d'un modèle de dépôt sédimentaire de la région sur la base des corrélations identifiées ; et/ou
(f) le procédé comprenant en outre le ciblage d'une sous-région de la région pour l'exploration d'hydrocarbures sur la base des corrélations identifiées, le procédé comprenant éventuellement le forage d'un puits latéral pour l'extraction d'hydrocarbures dans la sous-région ciblée.

6. Procédé selon l'une quelconque des revendications précédentes, les étapes des revendications 1, 5 (a), 5 (b), 5 (d), 5 (e) ou 5 (f) étant réalisées par un ordinateur.

7. Procédé comprenant la détermination de corrélations stratigraphiques entre deux ensembles ou plus de mesures stratigraphiques, chaque ensemble de mesures stratigraphiques étant obtenu à partir de différentes sections du même puits latéral dans une région :

(i) chaque ensemble de mesures stratigraphiques comprenant :

(a) des mesures d'un paramètre stratigraphique obtenu à partir d'une pluralité d'échantillons de roche provenant de la section respective du puits latéral, chaque échantillon de roche de la pluralité respective d'échantillons de roche étant associé à un emplacement de mesure différent à l'intérieur de la section respective du puits latéral ; et
(b) des mesures correspondantes d'un paramètre de profondeur pour chaque emplacement de mesure dans la section respective du puits latéral,

le paramètre stratigraphique étant un paramètre indicatif d'une caractéristique de la roche et le paramètre de profondeur étant un paramètre indicatif d'une profondeur souterraine d'un emplacement de mesure ; et

(ii) le procédé comprenant en outre, avant la détermination des corrélations stratigraphiques entre les deux ensembles ou plus de mesures stratigraphiques, la correction des deux ensembles ou plus de mesures stratigraphiques pour prendre en compte une inclinaison non nulle du puits latéral par rapport à la surface locale du sol,

le paramètre de profondeur mesurée pour chaque emplacement de mesure étant indicatif d'une profondeur souterraine dudit emplacement de mesure par rapport à la surface locale du sol, et la correction d'un ensemble de mesures stratigraphiques pour prendre en compte une inclinaison non nulle du puits latéral par rapport à la surface locale du sol comprenant :

la détermination de l'inclinaison du puits latéral par rapport à la surface locale du sol ; et
pour chaque emplacement de mesure dans le puits latéral, le calcul d'un paramètre de profondeur corrigé, indicatif d'une profondeur de l'emplacement de mesure par rapport à un horizon géologique local, compte tenu de l'inclinaison déterminée.

8. Procédé selon la revendication 7 :

(i) les différentes sections du même puits latéral étant des sections différentes d'un segment latéral du même puits latéral ;
(ii) les échantillons de roche étant des échantillons de déblais ; et/ou
(iii) le procédé comprenant en outre, avant la détermination de corrélations stratigraphiques entre les deux ensembles ou plus de mesures stratigraphiques, la mesure de valeurs du paramètre stratigraphique et de valeurs correspondantes du paramètre de profondeur, pour chacun de la pluralité d'échantillons de roche.

9. Procédé selon la revendication 7 ou la revendication 8, l'inclinaison du puits latéral étant l'inclinaison moyenne d'un segment latéral du puits latéral.

10. Procédé selon la revendication 9, l'inclinaison moyenne d'un segment latéral du puits latéral étant l'inclinaison d'un trajet en ligne droite moyen pour le segment latéral du puits latéral, la détermination de l'inclinaison moyenne du segment latéral du puits latéral comprenant éventuellement :

l'ajustement d'une droite à la trajectoire suivie par le segment latéral du puits latéral ; et
la détermination de l'inclinaison de la droite par rapport à la surface locale du sol ;
en outre, l'ajustement de la ligne droite et la détermination de l'inclinaison étant éventuellement réalisés en traçant le segment latéral du puits latéral en fonction (a) de la profondeur verticale au-dessous du niveau local du sol et (b) de la distance latérale parcourue le long du puits de forage et en ajustant une droite au segment latéral tracé.

11. Procédé selon l'une quelconque des revendications 7 à 10, les étapes de la revendication 7 étant réalisées par un ordinateur.

12. Programme informatique comprenant des instructions qui, lorsque le programme est exécuté par un ordinateur, amènent l'ordinateur à réaliser les étapes du procédé selon : les revendications 1, 5 (a), 5 (b), 5 (d), 5 (e) ou 5 (f) ; ou la revendication 7.

13. Support lisible par ordinateur stockant le programme informatique selon la revendication 12.

$P_A$

$D_A$

$P_B$

$D_B$

Fig. 1

Fig. 2

Fig. 3

Fig. 4

EP 4 176 258 B1

Fig. 5

Fig. 6

Fig. 7

Raw data

Fig. 8

Fig. 9 (a)

Fig. 9 (b)

Fig. 10

Fig. 11 (a)

Fig. 11 (b)

38

Fig. 11 (c)

Fig. 11 (d)

Fig. 11 (e)

Fig. 11 (f)

Fig. 11 (g)

Fig. 11 (h)

Fig. 12

Fig. 13

Fig. 14 (a)

EP 4 176 258 B1

Fig. 14 (b)

Fig. 14 (c)

Fig. 15

Fig. 16

Fig. 17

Fig. 18 (a)

Fig. 18 (b)

Fig. 19

Fig. 20 (a)

Fig. 20 (b)

Fig. 21

Fig. 22

Fig. 23

Fig. 24

Fig. 25

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016011384 A
- US 2016018556 A1
- WO 2015070022 A1

**Non-patent literature cited in the description**

- Fine reservoir structure modeling based upon 3D visualized stratigraphic correlation between horizontal wells: methodology and its application. **OU CHENGHUA et al.** JOURNAL OF GEOPHYSICS AND ENGINEERING. INSTITUTE OF PHYSICS PUBLISHING, 22 November 2017, vol. 14
- **MOHSEN ABDUL et al.** *Horizontal Well Correlation Using Real Time Data and Log Prediction in Geosteering Complex Reservoirs of Saudi Arabia*, 13 March 2013
- **EL GEZEERY T et al.** Innovative Geosteering Technology Utilized in Drilling Smart Multi-lateral Wells, Kuwait. *OFFSHORE TECHNOLOGY CONFERENCE*, 06 September 2013
- **MANN, KEITH OLIN**. Graphic Correlation. SEPM Society for Sedimentary Geology, 1995